# FASCICULE DE BREVET EUROPEEN

(11) **EP 0 868 184 B1**
(45) Date de publication et mention de la délivrance du brevet: **02.05.2003**
(21) Numéro de dépôt: 96943156.8
(22) Date de dépôt: 23.12.1996
(51) Int. Cl.: A61K 31/55, A61K 9/50

(54) **MICROGRANULES A LIBERATION PROLONGEE CONTENANT DU DILTIAZEM COMME PRINCIPE ACTIF**
DILTIAZEMHALTIGE MIKROGRANULATE MIT VERZÖGERTER WIRKSTOFFABGABE
SUSTAINED-RELEASE MICROGRANULES CONTAINING DILTIAZEM AS THE ACTIVE PRINCIPLE

(30) Priorité: 22.12.1995 FR 9515361
(43) Date de publication de la demande: 07.10.1998
(73) Titulaire: LABORATOIRES DES PRODUITS ETHIQUES ETHYPHARM, 78550 Houdan (FR)
(72) Inventeur: DEBREGEAS, Patrice, F-75007 Paris (FR); LEDUC, Gérard, F-45330 Malesherbes (FR); OURY, Pascal, F-75005 paris (FR); SUPLIE, Pascal, F-27400 Montaure (FR)
(74) Mandataire: Warcoin, Jacques
(86) Numéro de dépôt international: FR9602040
(87) Numéro de publication internationale: WO97023219

(56) Documents cités:
- EP-A- 0 149 920
- EP-A- 0 263 083
- EP-A- 0 318 398
- EP-A- 0 322 277
- WO-A-93/09767

## Description

La présente invention concerne de nouvelles formes de microgranules à libération prolongée (LP) contenant du diltiazem comme principe actif.

Le diltiazem est un dérivé de benzothiazépine antagoniste du calcium, utile pour le traitement de l'hypertension artérielle. Il peut être administré sous différentes formes, comprimés, solutions injectables ou gélules contenant des granules à libération prolongée. Ces dernières présentent l'avantage de permettre l'administration en une seule prise de la dose nécessaire à une journée de traitement.

Différentes formes de microgranules LP de diltiazem ont été décrites dans l'état de la technique, la plus intéressante étant celle décrite dans la demande de brevet EP-A-0 149 920, comprenant un noyau associant le diltiazem à un acide organique hydrosoluble, en particulier l'acide fumarique. En effet, le diltiazem, ou ses sels pharmaceutiquement acceptables sont peu solubles à pH neutre ou basique, et la présence d'un acide organique hydrosoluble s'est révélée particulièrement importante, permettant de créer un micro-environnement acide tamponné, favorisant à la fois la dilution et l'absorption du diltiazem dans des zones du tube digestif où le pH est trop élevé. Il a été néanmoins observé que pour ces formes LP, la solubilisation et l'absorption du diltiazem étaient dépendantes de l'absorption de nourriture, différentes lorsque la prise des microgranules est effectuée à jeun, ou pendant les repas.

Des microgranules LP sans acide organique ont été décrits dans la demande de brevet EP-A-0 613 370. Le noyau des microgranules est ici constitué par un grain neutre enrobé d'une succession de couches d'une part d'un polymère hydrosoluble et d'autre part de diltiazem.

Pour ces différentes formes, la libération prolongée du principe actif est assurée par une ou plusieurs couches d'enrobage du noyau des microgranules, associant généralement deux types de matériau polymère filmogène, l'un étant insoluble dans l'eau, le second étant hydrosoluble.

La présente invention concerne une nouvelle forme de microgranules LP contenant du diltiazem, ou un sel pharmaceutiquement acceptable de ce dernier, sans acide organique hydrosoluble, permettant d'obtenir une solubilisation et une absorption du principe actif au moins équivalente à celle obtenue en présence d'un acide. En outre, la présente invention concerne une nouvelle forme simple de microgranules LP contenant du diltiazem, de préparation aisée.

Les microgranules selon l'invention comprennent un support granulaire neutre, enrobé par une couche active comprenant du diltiazem, ou un sel pharmaceutiquement acceptable de ce dernier, un agent tensio-actif et un agent liant, et une couche assurant la libération prolongée du principe actif (ci-après couche LP).

Plus particulièrement, les microgranules conformes à la présente invention peuvent être de plusieurs catégories.

La première catégorie est représentée par des microgranules dont la couche LP assure une libération prolongée lente du principe actif.

La deuxième catégorie est représentée par des microgranules dont la couche LP assure une libération prolongée rapide du principe actif.

La différence entre ces deux catégories de microgranules monocouche réside essentiellement dans l'épaisseur de l'agent d'enrobage contenu dans la couche LP. En effet, plus cette couche LP est épaisse, plus la diffusion du principe actif sera lente.

Le profil de dissolution de chacun de ces deux types de microgranules est déterminé in vitro en utilisant l'eau comme milieu de dissolution et donne les spécifications suivantes :

### Microgranules rapides :

| Heures | Dissolution | Dissolution préférée |
|---|---|---|
| 2 H | 0 - 45 % | ≤ 30 % |
| 6 H | ≥ 50 % | ≥ 65 % |

### Microgranules lents :

| Heures | Dissolution | Dissolution préférée |
|---|---|---|
| 12 h | 0 - 60 % | ≤ 30 % |
| 20 H | ≥ 50 % | ≥ 45 % |

### Mélange :

| Heures | Dissolution | Dissolution préférée |
|---|---|---|
| 1H | ≤ 15 % | ≤ 15 % |
| 6H | ≤ 55 % | 25 - 55 % |
| 10H | 30 - 70 % | 30 - 60 % |
| 16 H | 30 - 85 % | 55 - 85 % |
| 22 H | - | ≥ 80 % |

La présente invention concerne également une troisième catégorie de microgranules résultant de l'enrobage de la couche LP assurant la libération prolongée lente du principe actif, c'est-à-dire de la couche LP des microgranules de la premère catégorie ci-dessus, d'une autre couche active comprenant :
. du diltiazem ou un sel pharmaceutiquement acceptable de ce dernier en tant que principe actif,
. un agent tensio-actif, et
. un agent liant,
elle-même enrobée d'une couche externe assurant une libération prolongée rapide du principe actif contenu dans cette couche active.

En d'autres termes, ces microgranules dits "double couche" sont constitués, du centre vers la périphérie, d'un support granulaire neutre, d'une première couche active, d'une couche LP assurant la libération prolongée lente du principe actif contenu dans la première couche active, d'une seconde couche active et d'une couche LP assurant la libération prolongée rapide du principe actif contenu dans la seconde couche active.

Il est entendu que pour les microgranules dits "double-couche", seules les spécifications décrites ci-dessus et correspondant aux microgranules lents et mélanges, peuvent être appliquées.

Avantageusement, une couche dite d'enrobage protecteur ou intercalaire est appliquée sur le microgranule entre la couche LP assurant la libération prolongée lente du principe actif et la seconde couche de principe actif.

Il a en effet été constaté que lors de l'application de la seconde couche de diltiazem, et/ou lors de la dissolution des microgranules, il s'établit des interactions entre le diltiazem et la couche LP située entre les deux couches actives, ceci entrainant la modification de la libération prolongée du diltiazem contenu dans la première couche active. Cette couche intercalaire joue en quelque sorte le rôle d'un isolant entre les deux couches concernées, les protégeant l'une de l'autre. Un autre moyen d'obtenir le même résultât consisterait à utiliser pour la couche LP en question un agent d'enrobage n'interagissant pas avec le diltiazem.

Le support granulaire neutre est constitué par tout support granulaire neutre pharmaceutiquement acceptable, d'un diamètre moyen compris entre 0,4 et 0,9 mm, de préférence 0,4 et 0,6 mm. Il s'agit de préférence de grains constitués de saccharose et d'amidon dans un rapport pondéral voisin de 75/25, tels que ceux décrits sous la dénomination de "Sugar Spheres" (Handbook of Pharmaceutical Excipients, 2de Ed., The Pharmaceutical Press, London, 1994).

Parmi les sels pharmaceutiquement acceptables du diltiazem, on trouve de préférence le chlorhydrate.

L'agent tensio-actif peut être un tensio-actif anionique, non-ionique, cationique ou amphotère. D'une manière préférentielle, il s'agit d'un tensio-actif anionique. Parmi les tensio-actifs anioniques, on trouve les alkylsulfates de métal alcalin en C₁₀-C₂₀, de préférence le laurylsulfate de sodium, les alkylsulfonates de métal alcalin en C₁₀-C₂₀, ou encore les alkyl-benzènesulfonates de métal alcalin en C₁₀-C₂₀. Par métal alcalin on entend de préférence le sodium ou le potassium.

L'agent liant est constitué par tout agent liant pharmaceutiquement acceptable, utile pour l'enrobage des supports granulaires neutres, en particulier des polymères pharmaceutiquement acceptables tels que les polyvinylpyrrolidones décrites sous le nom de povidone (Handbook of Pharmaceutical Excipients, 2de Ed., The Pharmaceutical Press, London, 1994), ou les hydroxypropyl méthyl celluloses (HPMC), les polyéthylèneglycols (PEG), etc.

La couche active comprenant le diltiazem peut également comprendre d'autres additifs usuels, tels qu'un agent plastifiant.

D'une manière générale, le rapport pondéral principe actif / support granulaire neutre est voisin de 4/1. Par principe actif, on entend le diltiazem ou les sels pharmaceutiquement acceptables de ce dernier.

D'une manière avantageuse, le rapport pondéral principe actif / agent tensio-actif est compris entre 99/1 et 95/5, de préférence d'environ 98/2.

Le rapport pondéral principe actif / agent liant est pour sa part compris entre 99/1 et 90/10, de préférence voisin de 97/3.

De préférence, les microgranules monocouche selon l'invention comprennent pour le support et la couche active les éléments suivants, les pourcentages étant donnés en poids pour un total de 100 :
- support granulaire neutre 20 - 25 %
- principe actif 70 - 75 %
- agent tensio-actif 0,5 - 5 %
- agent liant 0,5 - 10 %
- agent plastifiant 0 - 5 %.

De préférence, les microgranules double couche selon l'invention comprennent pour le support et les couches actives les éléments suivants, les pourcentages étant donnés en poids pour un total de 100 :
- support granulaire neutre 10 - 20 %
- principe actif 75 - 85 %
- agent tensio-actif 0,5 - 5 %
- agent liant 0,5 - 10 %
- agent plastifiant 0,5 - 5 %.

Chaque couche LP est constituée par un agent d'enrobage assurant la libération prolongée, éventuellement associé à un ou plusieurs additifs usuels, en particulier un adjuvant de biodisponibilité et/ou un agent plastifiant et/ou un agent lubrifiant.

De préférence, l'agent d'enrobage assurant la libération prolongée est un polymère filmogène insoluble dans l'eau, tel que les polyméthacrylates (Handbook of Pharmaceutical Excipients, 2de Ed., The Pharmaceutical Press, London, 1994), en particulier les poly(éthyl acrylate, méthyl méthacrylate, chlorure de triméthylammonioéthyl méthacrylate), commercialisés sous la marque Eudragit® RS.

L'adjuvant de biodisponibilité est de préférence un ester d'acide gras et de polyoxuéthylène, en particulier ceux décrits sous le nom de polysorbate (Handbook of Pharmaceutical Excipients, 2de Ed., The Pharmaceutical Press, London, 1994), en particulier commercialisés sous la marque Montanox®.

L'agent lubrifiant est constitué par un lubrifiant usuel pharmaceutiquement acceptable, employé dans la préparation de microgranules, en particulier du talc.

L'agent plastifiant est un agent plastifiant usuel pharmaceutiquement acceptable employé pour la préparation de microgranules, en particulier les esters des acides citrique, phtalique et sébacique, en particulier les esters aliphatiques tels que le citrate de triéthyle, le sébaçate de dibutyle ou le phtalate de diéthyle, et leurs mélanges.

D'une manière préférentielle, on emploie pour chaque couche active un ester de l'acide phtalique, et pour chaque couche LP un mélange des esters des acides citrique et sébacique.

La couche d'enrobage protecteur ou intercalaire, quand elle existe, est constituée par un polymère type méthacrylique, un plastifiant, un lubrifiant et éventuellement un adjuvant de biodisponibilité.

D'une manière avantageuse, les microgranules monocouche selon l'invention ont la composition finale suivante, les pourcentages étant exprimés en poids pour un total de 100 %:
- support granulaire neutre 10 - 20 %
   - couche active :
      - principe actif 45 - 65 %
      - agent liant 0,5 - 2 %
      - agent tensio-actif 0,5 - 1 %
      - agent plastifiant 0,5 - 1 %
   - couche LP :
      - agent d'enrobage 10 - 30 %
      - adjuvant de biodisponibilité 0,05 - 0,15 %
      - agent plastifiant 2 - 10 %
      - agent lubrifiant 2 - 10 %.

      D'une manière avantageuse, les microgranules double couche selon l'invention ont la composition finale suivante, les pourcentages étant exprimés en poids sec pour un total de 100 % :
      - support granulaire neutre 10 - 20 %
   - couches actives :
      - principe actif 45 - 60 %
      - agent liant 0,5 - 2 %
      - agent tensio-actif 0,5 - 1 %
      - agent plastifiant 0,5 - 1 %
   - couches LP :
      - agent d'enrobage 10 - 30 %
      - adjuvant de biodisponibilité 0,05 - 0,15 %
      - agent plastifiant 2 - 10 %
      - agent lubrifiant 2 - 10 %.

La teneur en principe actif de chaque couche active peut être identique ou différente selon la vitesse et la quantité de principe actif que l'on souhaite libérer au cours du temps. Les adaptations nécessaires en fonction du but à atteindre sont à la portée de tout homme du métier.

Les microgranules selon l'invention sont préparés selon les techniques usuelles par montage du principe actif sur le support granulaire neutre, puis enrobage par la couche LP, l'opération étant renouvelée pour les microgranules double couche.

Le montage du principe actif est effectué par pulvérisation discontinue d'une solution hydroalcoolique contenant l'agent liant, l'agent tensio-actif, et éventuellement l'agent plastifiant, en alternance avec des séquences de poudrage du principe actif et des séquences de repos.

Les microgranules de diltiazem ainsi obtenus sont ensuite tamisés et séchés.

L'enrobage par chaque couche LP est ensuite effectué par pulvérisation d'une suspension aqueuse contenant l'agent d'enrobage et les additifs usuels. Les microgranules LP ainsi obtenus sont ensuite tamisés et séchés.

Cette opération d'enrobage par chaque couche LP est répétée le nombre de fois nécessaire à l'obtention de la cinétique de libération désirée.

D'autres caractéristiques des microgranules selon l'invention apparaîtront à la lecture des exemples ci-après dans lesquels on utilise des turbines en rotation à titre préférentiel, tout autre appareil d'enrobage, en particulier à lit fluidisé du type GLATT ou NIRO, pouvant également être utilisé avec les transpositions usuelles que l'homme du métier connaît.

### Exemple 1 : Préparation de microgranules

### 1.1 Préparation de la solution de montage

On pèse les excipients de montage dans les proportions suivantes :

| | |
|---|---|
| PVP K 17*(povidone) | 50 % du VS total |
| Lauryl sulfate de sodium | 25 % du VS total |
| Phtalate de diéthyl | 25 % du VS total |
| Eau purifiée | 50 % du S total |
| Alcool éthylique à 95 % | 50 % du S total |

| | |
|---|---|
| * commercialisé par la société BASF | |

Dans un premier mélangeur inox, on verse l'alcool éthylique à 95 % puis on incorpore par petites quantités le PVP K 17 sous agitation. On maintient l'agitation jusqu'à l'homogénéité de la solution. On obtient un solution alcoolique à 15 % de PVP K 17.

Dans un second mélangeur inox, on verse l'eau purifiée puis on incorpore par petites quantités sous agitation le lauryl sulfate de sodium. On maintient l'agitation jusqu'à l'homogénéité de la solution. On obtient une solution aqueuse à 7,5 % de lauryl sulfate de sodium.

On mélange ensuite les deux solutions ci-dessus et on ajoute sous agitation le phtalate de diéthyle.

### 1.2 Montage du diltiazem

On place les grains support (Sugar Spheres) dans une turbine à dragéification en rotation. On effectue le montage du principe actif (chlorhydrate) sur les grains support par pulvérisation discontinue de la suspension de montage obtenue ci-dessus, en alternance avec des séquences de poudrage du principe actif et des séquences de repos. L'opération de poudrage est répétée jusqu'à obtention de la teneur en principe actif désirée. On tamise les microgranules obtenus, puis on les sèche.

On obtient des microgranules à libération immédiate de composition suivante, les pourcentages étant donnés en poids :

| | |
|---|---|
| diltiazem | 73,05 % |
| grains support | 22,55 % |
| PVP K 17 | 2.20 % |
| lauryl sulfate de sodium | 1,10 % |
| phtalate de diéthyl | 1,10 % |

pour une teneur en diltiazem (chlorhydrate) de 735,75 mg/g de microgranules.

### 1.3 Préparation de la solution d'enrobage

On pèse les excipients d'enrobage suivants dans les proportions indiquées :

| | |
|---|---|
| Eudragit RS 30 D* | VS∗ = 30,0 % de la masse d'Eudragit pesée |
| sébaçate de dibutyl | VS = 4,0 % du VS d'Eudragit |
| Montanox 80 DF** | VS = 0,4 % du VS d'Eudragit |
| Talc | VS = 20,0 % du VS d'Eudragit |
| citrate de triéthyl | VS = 16,0 % du VS d'Eudragit |
| eau purifiée | S°= 50,0 % de la masse d'Eudragit pesée |

| | |
|---|---|
| * commercialisé par la société Rhöm Pharma | |
| VS∗ = vernis sec ou extrait sec (ce qui reste après évaporation des solvants) | |
| ** distribué par la société Seppic | |
| S° = solvant dans lequel les excipients sont dissous ou dilués. | |

Dans un bécher inox, on verse l'eau purifiée, puis on incorpore par petites quantités sous agitation le Montanox 80 DF, le citrate de triéthyl puis le sébaçate de dibutyl. On maintient l'agitation jusqu'à homogénéité de la solution, puis on incorpore le talc par petites quantités sous agitation. Enfin, on ajoute l'Eudragit RS 30D en maintenant l'agitation jusqu'à homogénéité de la suspension, puis pendant toute la durée de la phase d'enrobage.

### 1.4 Enrobage des microgranules

On place les microgranules obtenus précédemment dans une turbine à dragéification en rotation, puis on effectue l'enrobage des microgranules par pulvérisation continue de la suspension obtenue ci-dessus. On tamise la masse de microgranules enrobés obtenue, puis on les sèche en turbine en rotation à température ambiante, ou par soufflage d'air froid si la température ambiante est supérieure à 24°C.

On répète cette suite d'opérations le nombre de fois nécessaire à l'obtention de la cinétique désirée.

On lubrifie enfin les microgranules LP obtenus par l'ajout de talc lors de la phase de mélange.

### Exemple 2

Par la mise en oeuvre du procédé décrit dans l'exemple 1, en faisant varier la quantité d'Eudragit RS 30 D et d'excipients, on obtient les microgranules de compositions décrites dans le Tableau 1 ci-après.

**Tableau I**

| % | | 1 | | 2 | | 3 | | 4 | | 5 | | 6 | | 7 | | 8 | | 9 |
|---|---|---|---|---|---|---|---|---|---|---|---|---|---|---|---|---|---|---|
| **PA** | | 55,56 | | 62,17 | | 51,92 | | 55,81 | | 61,49 | | 45,1 | | 50,37 | | 50,50 | | 54,12 |
| **N 30** | | 17,08 | | 19,19 | | 16,03 | | 17,23 | | 18,98 | | 13,92 | | 15,55 | | 15,59 | | 16,71 |
| **PVP** | | 1,86 | | 1,87 | | 1,56 | | 1,98 | | 1,85 | | 1,36 | | 1,52 | | 1,52 | | 1,63 |
| **LAS** | | 0,93 | | 0,94 | | 0,78 | | 0,84 | | 0,93 | | 0,68 | | 0,76 | | 0,76 | | 0,81 |
| **DP** | | 0,93 | | 0,94 | | 0,78 | | 0,84 | | 0,93 | | 0,68 | | 0,76 | | 0,76 | | 0,81 |
| **E RS** | | 16,83 | | 10,59 | | 20,60 | | 16,45 | | 11,27 | | 27,19 | | 21,72 | | 21,98 | | 18,10 |
| **DS** | | 2,73 | | 0,42 | | 0,83 | | 0,66 | | 0,44 | | 1,12 | | 0,88 | | 0,88 | | 0,72 |
| **M 80** | | 0,07 | | 0,05 | | 0,08 | | 0,07 | | 0,05 | | 0,11 | | 0,09 | | 0,09 | | 0,08 |
| **Talc** | | 2,85 | | 2,13 | | 4,12 | | 3,79 | | 2,25 | | 5,47 | | 4,86 | | 4,39 | | 4,11 |
| **TC** | | 1,15 | | 1,71 | | 3,31 | | 2,64 | | 1,81 | | 4,37 | | 3,49 | | 3,53 | | 2,91 |
| **Total** | 100 % | | | | | | | | | | | | | | | | | |
| **mg/g** | | 555,63 | | 599,55 | | 504,51 | | 516,05 | | 602,03 | | 452,62 | | 497,29 | | 482,36 | | 536,31 |

Les pourcentages sont donnés en poids sec de microgranules, avec les abréviations suivantes :
PA = principe actif - chlorhydrate de diltiazem ;
N 30 = Neutres 30 ;
PVP = PVP K 17 ;
LAS = lauryl sulfate de sodium ;
DP = phtalate de diéthyl ;
E RS = Eudragit RS 30 D ;
DS = sébaçate de dibutyl ;
M 80 = Montanox 80 DF ;
TC = citrate de triéthyl ;
mg/g = teneur en diltiazem en mg par g de microgranules LP.

Les microgranules 4 sont constitués par un mélange des microgranules 2 et 3, lubrifiés avec 0,5 % de talc (% = poids de talc / poids des microgranules à lubrifier).

Les microgranules 5 sont obtenus par un second enrobage de 400 g des microgranules 2 avec la suspension de l'exemple 1.1.

Les microgranules 6 sont obtenus par un second enrobage de 500 g des microgranules 2 avec la suspension de l'exemple 1.1.

Les microgranules 7 sont constitués par un mélange des microgranules 5 et 6, lubrifiés avec 0,5 % de talc.

Les microgranules 8 sont obtenus par un second enrobage de 400 g des microgranules 2 avec la suspension de l'exemple 1.1.

Les microgranules 9 sont constitués par un mélange des microgranules 5 et 8, lubrifiés avec 0,5 % de talc.

### Exemple 3 : Cinétiques de dissolution

Les microgranules ci-dessus sont soumis à une étude cinétique de dissolution dans l'eau sur 24 heures. Les résultats obtenus sont résumés dans le Tableau II ci-après, exprimés en pourcentage de principe actif en solution par rapport au total de principe actif.

**Tableau II**

| **t(h)** | **1** | **2** | **3** | **4** | **5** | **6** | **7** | **8** | **9** |
|---|---|---|---|---|---|---|---|---|---|
| 2 | 2,2 | 12,7 | 0,8 | 4,9 | 3,1 | 1,2 | 2,1 | 1,1 | 1,7 |
| 4 | 29,6 | 82,9 | 1,1 | 36,4 | 37,0 | 1,4 | 19,9 | 1,7 | 20,2 |
| 6 | - | 94,1 | 1,4 | 41,1 | 85,7 | 1,9 | 38,0 | 1,8 | 36,5 |
| 8 | - | 97,2 | 1,9 | 42,2 | 90,8 | 2,3 | 39,9 | 2,1 | 38,2 |
| 10 | - | 98,8 | 3,8 | 43,6 | 92,8 | 2,6 | 40,9 | 2,5 | 39,0 |
| 12 | 50,6 | 100 | 30,9 | 56,2 | 94,1 | 3,0 | 41,5 | 3,2 | 39,7 |
| 14 | 63,3 | - | 74,8 | 82,8 | 94,9 | 3,4 | 42,0 | 18,7 | 45,5 |
| 16 | - | - | 88,9 | 92,0 | 95,4 | 4,4 | 43,6 | 63,7 | 68,3 |
| 18 | 94,4 | - | 93,7 | 95,0 | 95,7 | 12,5 | 50,7 | 84,6 | 83,4 |
| 20 | - | - | 96,5 | 96,7 | 96,0 | 55,7 | 65,9 | 91,0 | 88,1 |
| 22 | - | - | - | 97,7 | 96,1 | 85,2 | 80,7 | 94,4 | 90,3 |
| 24 | 98,0 | - | - | 98,7 | - | - | 92,3 | - | 91,5 |

### Exemple 4 : Bioéquivalence avec le Cardizem® CD

La bioéquivalence de gélules dosées à 300 mg de diltiazem contenant les microgranules de composition 9 (Diltiazem CD) a été comparée in vivo au Cardizem® CD 300 mg commercialisé par la société Marion Merrell Dow Inc.aux Etats-Unis.

Les études ont été menées sur trois groupes de 12 patients, par mesure de la concentration plasmatique en principe actif.

Pour le premier groupe, le Cardizem® CD a été administré à jeun (référence).

Pour le second groupe, le Diltiazem CD a été administré à jeun ("test-fast").

Pour le troisième groupe, le Diltiazem CD a été administré avec administration concomitante d'un repas standardisé, le "breakfeast" américain ("rest-fed").

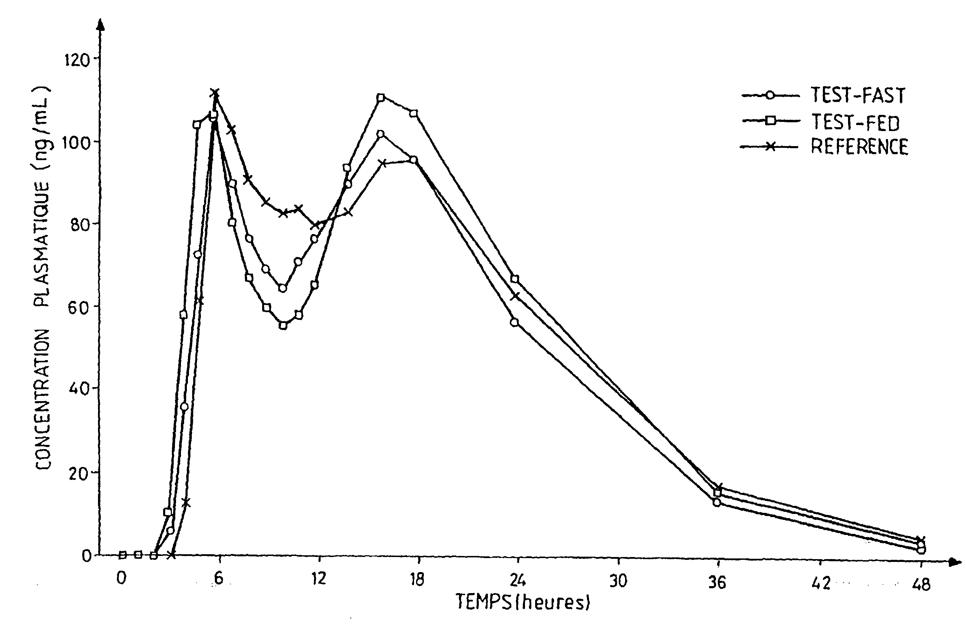

Les courbes des concentrations plasmatiques moyennes sont reportées sur la figure 1 en annexe.

Cette étude conclut sans équivoque à la bioéquivalence du Diltiazem CD selon l'invention avec le Cardizem® CD, indépendamment de la prise concomitante de nourriture.

### Exemple 5 : Préparation de microgranules double couche

5.1 - Microgranules double couche sans solution de prémontage (lot YED 006x1383.1)

### A) Montage de la première couche de Diltiazem, réalisation du lot YED 006.

a) Préparation de la solution de montage
• Proportions des excipients mis en oeuvre :

| | |
|---|---|
| PVP K 17 | 50 % du VS∗ total |
| LAURYL SULFATE DE SODIUM | 25 % du VS total |
| DIETHYL PHTALATE | 25 % du VS total |
| EAU PURIFIEE | 50 % du S° total |
| ALCOOL ETHYLIQUE 95 % | 50 % du S total |

| | |
|---|---|
| VS∗ = vernis sec ou extrait sec (ce qui reste après évaporation des solvants). | |
| S° = solvants dans lesquels les excipients sont dissous ou dilués. | |

• La solution est préparée dans un récipient inox,
• L'ALCOOL ETHYLIQUE 95 % puis l'EAU PURIFIEE sont versés dans le récipient puis mis en agitation,
• Le PVP K 17 puis le LAURYL SULFATE sont incorporés successivement et par petites quantités,
• L'agitation est maintenue jusqu'à complète dissolution du PVP K 17 et du LAURYL SULFATE,
• Le DIETHYL PHTALATE est ensuite ajouté et, l'agitation est maintenue jusqu'à homogénéité de la solution.
b) Montage du DILTIAZEM sur les grains supports neutres
- Des grains support NEUTRES 30 sont placés dans une turbine à dragéification en rotation,
- Le montage du principe actif est effectué sur les NEUTRES 30, par pulvérisation discontinue de la suspension décrite ci-dessus en alternance avec des séquences de poudrage du DILTIAZEM, et des séquences de repos,
- La masse de microgranules obtenue est tamisée sur une grille d'ouverture de maille allant de 0,85 à 1,18 mm,
- Les microgranules sont ensuite séchés dans la turbine en rotation
   * à température ambiante et pendant 2H30 entre deux phases consécutives.
   * à 35°C et pendant 4 à 6H pour la dernière phase de montage de chaque journée. A l'issue du séchage, la turbine est laissée en rotation 4H sans apport de chaleur.
- La masse obtenue est ensuite lubrifiée avec du TALC.

c) Formule des microgranules après montage de la première couche de Diltiazem :

| Composition du VS∗ | Quantité en % |
|---|---|
| DILTIAZEM | 74,17 |
| NEUTRES 30 | 21,26 |
| PVP K 17 | 2,16 |
| LAURYL SULFATE DE SODIUM | 1,08 |
| DIETHYL PHTALATE | 1,08 |
| TALC | 0,24 |
| Teneur théorique | 741,7 mg/g |
| Teneur obtenue | 735,9 mg/g |

| | |
|---|---|
| VS∗ = vernis sec ou extrait sec (ce qui reste après évaporation des solvants). | |

### B) Enrobage interne du lot YED 006, réalisation du lot YED006x1383

a) Préparation de la suspension d'enrobage
• Proportions des excipients mis en oeuvre :

| | |
|---|---|
| AQUACOAT ECD 30 | VS∗ = 30,0 % de la masse d'AQUACOAT pesée |
| DIBUTYL SEBAÇATE | VS = 24,0 % du VS d'AQUACOAT |

| | |
|---|---|
| VS∗ = vernis sec ou extrait sec (ce qui reste après évaporation des solvants). | |

• La suspension est préparée dans un récipient inox dans lequel on introduit l'AQUACOAT ECD 30,
• Le DIBUTYL SEBAÇATE est incorporé par petites quantités à AQUACOAT mis en agitation,
• L'agitation est maintenue jusqu'à homogénéité de la suspension (environ 15 minutes).
b) Enrobage interne des microgranules de DILTIAZEM
- Les microgranules à enrober (issus du lot YED 006) sont placés dans une turbine à dragéification,
- L'enrobage des microgranules est effectué par pulvérisation continue de la suspension décrite ci-dessus,
- La masse de microgranules obtenue est tamisée sur une grille d'ouverture de maille de 1,25 mm,
- Les microgranules sont ensuite séchés en turbine en rotation à une température de 40°C pendant 2H puis pendant 7H à température ambiante.

c) Formule du lot YED006x1383 après enrobage interne

| Composition du VS∗ | Quantité en % |
|---|---|
| DILTIAZEM | 56,61 % |
| NEUTRES 30 | 16,22 % |
| PVP K 17 | 1,65 % |
| LAURYL SULFATE DE SODIUM | 0,83 % |
| DIETHYL PHTALATE | 0,83 % |
| AQUACOAT BCD 30 | 19,10 % |
| DIBUTYL SEBAÇATE | 5,59 % |
| TALC | 0,18 % |
| Teneur Théorique | 566,1 mg/g |

| | |
|---|---|
| VS∗ = vernis sec ou extrait sec (ce qui reste après évaporation des solvants). | |

### C) Montage de la seconde couche de DILTIAZEM sur le lot YED006x1383, réalisation du lot YED 006x1383.1

a) Préparation de la solution de montage
- Se reporter au paragraphe A)a).

b) Montage du DILTIAZEM
- Le montage du principe actif est effectué sur les microgranules issus de l'enrobage du lot YED006x1383, par pulvérisation discontinue de la suspension décrite ci-dessus en alternance avec des séquences de poudrage du DILTIAZEM, et des séquences de repos,
- La masse de microgranules obtenue est tamisée sur une grille d'ouverture de maille de 1,50 mm,
- Les microgranules sont ensuite séchés dans la turbine en rotation à température ambiante et ce, pendant 8H.

c) Formule du lot YED006x1383.1 après montage de la seconde couche de DILTIAZEM

| Composition du VS∗ | Quantité en % |
|---|---|
| DILTIAZEM | 67,14 % |
| NEUTRES 30 | 11,54 % |
| PVP 17 | 2,17 % |
| LAURYL SULFATE DE SODIUM | 1,09 % |
| DIETHYL PHTALATE | 1,09 % |
| AQUACOAT ECD 30 | 13,59 % |
| DIBUTYL SEBAÇATE | 3,26 % |
| TALC | 0,13 % |
| Teneur Théorique | 674,1 mg/g |

| | |
|---|---|
| VS∗ = vernis sec ou extrait sec (ce qui reste après évaporation des solvants). | |

### D) Enrobage externe du lot YED006x1383.1

a) Préparation de la suspension d'enrobage
• Proportions des excipients mis en oeuvre :

| | |
|---|---|
| EUDRAGIT RS 30 D | VS∗= 30,0 % de la masse d'EUDRAGIT pesée |
| DIBUTYL SEBACATE | VS = 4,0 % du VS d'EUDRAGIT |
| POLYSORBATE 80 | VS = 0,4 % du VS d'EUDRAGIT |
| TRIETHYL CITRATE | VS = 16,0 % du VS d'EUDRAGIT |
| TALC | VS = 10,0 % du VS d'EUDRAGIT |
| EAU PURIFIEE | S° = 50,0 % de la masse d'EUDRAGIT pesée |

| | |
|---|---|
| VS∗ = vernis sec ou extrait sec (ce qui reste après évaporation des solvants). | |
| S° = solvant dans lequel les excipients sont dissous ou dilués. | |

• La suspension est préparée dans un récipient inox dans lequel on introduit l'EAU PURIFIEE,
• Le POLYSORBATE 80, le TRIETHYL CITRATE, et le DIBUTYL SEBAÇATE (USP) sont incorporés successivement et par petites quantités à l'EAU PURIFIEE mise en agitation,
• L'agitation est maintenue jusqu'à homogénéité de la solution (environ 15 minutes),
• L'EUDRAGIT RS 30 D et le talc est ensuite ajouté,
• L'agitation est maintenue jusqu'à homogénéité du mélange (environ 15 minutes), puis durant tout l'enrobage.
b ) Enrobage externe des microgranules de DILTIAZEM
- Les microgranules à enrober sont placés dans une turbine à dragéification,
- L'enrobage des microgranules est effectué par pulvérisation continue de la suspension décrite ci-dessus,
- La masse de microgranules obtenue est tamisée sur une grille d'ouverture de maille de 1,40 mm,
- Les microgranules sont ensuite séchés en turbine en rotation à une température de 40°C pendant 1H puis 8H à 30°C.

c) Formule du lot YED 006X1383.1 après enrobage externe

| Composition du VS∗ | Quantité en % |
|---|---|
| DILTIAZEM | 58,99 % |
| NEUTRES 30 | 10,14 % |
| PVP K 17 | 1,91 % |
| LAURYL SULFATE DE SODIUM | 0,95 % |
| DIETHYL PHTALATE | 0,95 % |
| EUDRAGIT RS 30 D | 8,62 % |
| AQUACOAT ECD 30 | 11,94 % |
| DIBUTYL SEBAÇATE | 3,22 % |
| POLYSORBATE 80 | 0.04 % |
| TALC | 1,84 % |
| TRIETHYL CITRATE | 1,40 % |
| Teneur Théorique | 589,9 mg/g |

| | |
|---|---|
| VS∗ = vernis sec ou extrait sec (ce qui reste après évaporation des solvants). | |

### E) Résultats de dissolution après l'enrobage interne, le montage de la seconde couche de Diltiazem, et l'enrobage externe

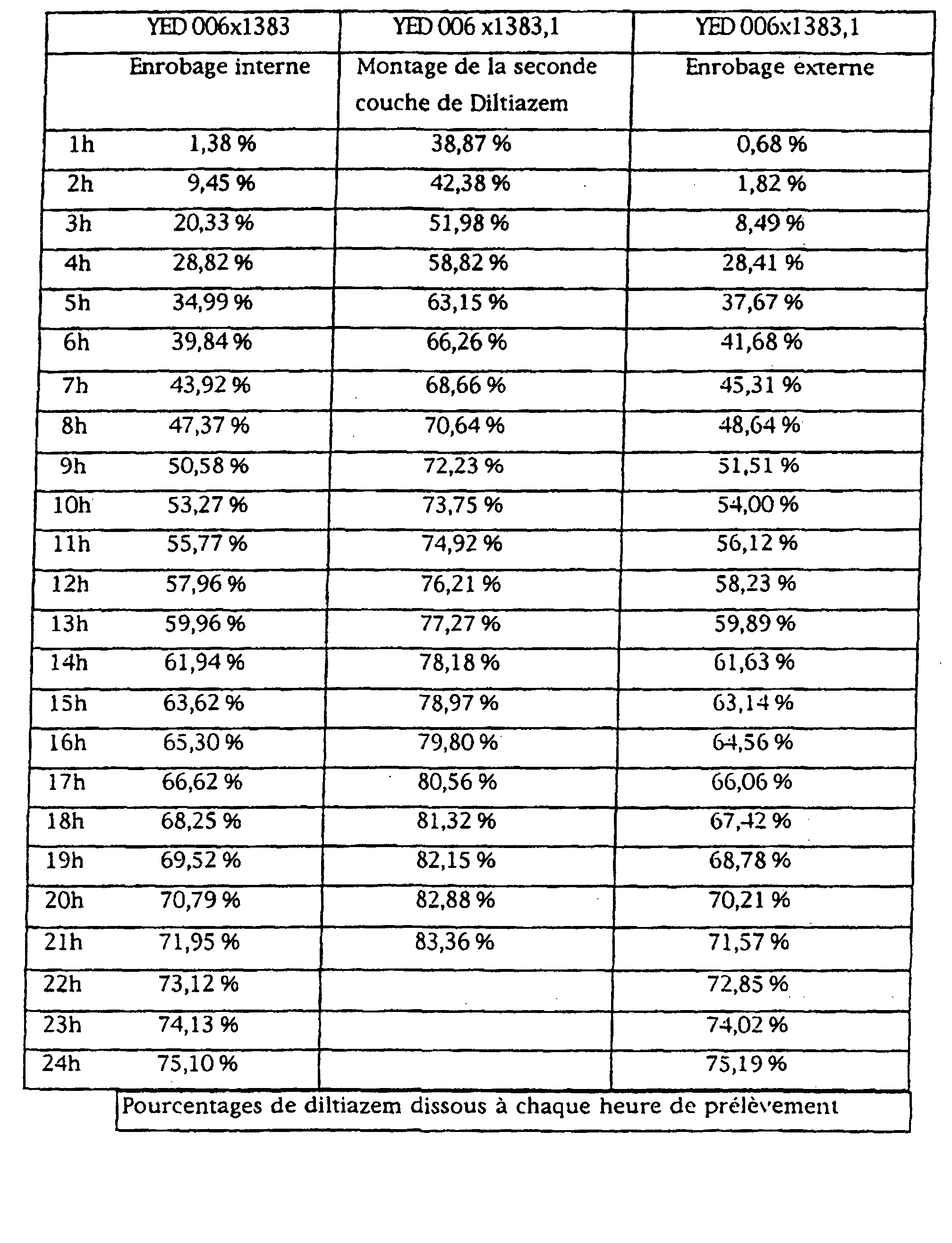

### F) Formules du lot YED 006 x 1383.1

- pourcentages donnés en poids pour un total de 100 (hors enrobage) :

| | **YED 006 x 1383.1** |
|---|---|
| Montage | |
| Principe actif | 80.87 % |
| Support Neutre | 13.90 % |
| Agent liant | 2.61 % |
| Tensio-actif | 1.31 % |
| Plastifiant | 1.31 % |

- pourcentage en poids sec pour un total de 100 % (composition finale) :

| | **YED 006 x 1383.1** |
|---|---|
| Support neutre | 10.14 % |
| Montage | |
| Principe actif | 58.99 % |
| Agent liant | 1.91 % |
| Tensio-actif | 0.95 % |
| Plastifiant | 0.95 % |
| Enrobage | |
| Agent d'enrobage | 20.56 % |
| Adjuvant de biodisponibilité | 0.04 % |
| Plastifiant | 4.62 % |
| Lubrifiant | 1.84 % |

### 5.2 - Microgranules double couche avec solution d'enrobage protecteur

### I - Lot YED 005Bx1350

### A) Montage de la première couche de Diltiazem, réalisation du lot YED 005

a) Préparation de la solution de montage
- Se reporter au paragraphe 5.1 A) a).

b) Montage du DILTIAZEM sur les grains supports neutres
- Se reporter au paragraphe 5.1 A) b).

c) Formule du lot YED 005 après montage

| Composition du VS∗ | Quantité en % |
|---|---|
| DILTIAZEM | 74,65 % |
| NEUTRES 30 | 21,37 % |
| PVP K 17 | 1,99 % |
| LAURYL SULFATE DE SODIUM | 1,00 % |
| DIETHYL PHTALATE | 1,00 % |
| Teneur théorique | 746,50 mg/g |
| Teneur obtenue | 711,00 mg/g |

| | |
|---|---|
| VS∗ = vernis sec ou extrait sec (ce qui reste après évaporation des solvants). | |

### B) Enrobage interne du lot YED 005, réalisation du lot YED 005B

a) Préparation de la suspension d'enrobage
• Proportions des excipients mis en oeuvre :

| | |
|---|---|
| EUDRAGIT RS 30 D | VS∗ = 30,0 % de la masse d'EUDRAGIT pesée |
| DIBUTYL SEBAÇATE | VS = 4,0 % du VS D'EUDRAGIT |
| POLYSORBATE 80 | VS = 0,4 % du VS d'EUDRAGIT |
| TALC | VS = 10,0 % du VS d'EUDRAGIT |
| TRIETHYL CITRATE | VS = 16,0 % du VS d'EUDRAGIT |
| EAU PURIFIEE | S°= 50,0 % de la masse d'EUDRAGIT pesée |

| | |
|---|---|
| VS∗ = vernis sec ou extrait sec (ce qui reste après évaporation des solvants). | |
| S ° = solvant dans lequel les excipients sont dissous ou dilués. | |

• La suspension est préparée dans un récipient inox dans lequel on introduit l'EAU PURIFIEE,
• Le POLYSORBATE 80, le TRIETHYL CITRATE, et le DIBUTYL SEBAÇATE (USP) sont incorporés successivement et par petites quantités à l'EAU PURIFIEE mise en agitation,
• L'agitation est maintenue jusqu'à homogénéité de la solution (environ 15 minutes),
• L'EUDRAGIT RS 30 D est ensuite ajouté,
• Le TALC est incorporé par petites quantités à la suspension,
• L'agitation est maintenue jusqu'à homogénéité du mélange (environ 15 minutes), puis durant tout l'enrobage,
• Une quantité de TALC équivalente à celle mise en suspension sera préparée afin d'être poudrée sur la masse durant l'enrobage,
b) Enrobage interne des microgranules de DILTIAZEM
- Les microgranules à enrober (issus du lot YED 005) sont placés dans une turbine perforée,
- L'enrobage des microgranules est effectué à une température de 30°C par pulvérisation continue de la suspension décrite ci-dessus, en alternance avec des séquences de poudrage de TALC (quantité identique à celle mise en suspension),
- La masse de microgranules obtenue est tamisée sur une grille d'ouverture de maille allant de 1,18 à 1,25 mm,
- Les microgranules sont ensuite séchés en turbine en rotation à une température de 30°C pendant 1H puis 40°C pendant 2H,
- La température est ramenée à 30°C avant de procéder à un second tamisage sur une grille d'ouverture de maille allant de 1,18 à 1,25 mm,
- Les microgranules sont ensuite replacés en turbine en rotation à une température de 30°C et ce, pendant tout l'intervalle de temps entre la phase d'enrobage achevée et la suivante,
- Cette suite d'opération est reproduite jusqu'à obtention de la cinétique désirée,
- A l'issue de l'étape d'enrobage, les microgranules sont tamisés sur une grille d'ouverture de maille de 0,60 mm,
- Ils sont ensuite lubrifiés avec une quantité de TALC équivalente à 0,75 % de la masse enrobée obtenue.

c) Formule du lot YED 005B après enrobage interne

| Composition du VS∗ | Quantité en % |
|---|---|
| DILTIAZEM | 53,20 % |
| NEUTRES 30 | 15,23 % |
| PVP K 17 | 1,42 % |
| LAURYL SULFATE DE SODIUM | 0,71 % |
| DIETHYL PHTALATE | 0,71 % |
| EUDRAGIT RS 30 D | 19,96 % |
| DIBUTYL SEBAÇATE | 0,80 % |
| POLYSORBATE 80 | 0,08 % |
| TALC | 4,70 % |
| TRIETHYL CITRATE | 3,19 % |
| Teneur Théorique | 532,0 mg/g |
| Teneur Trouvée | 537,3 mg/g |

| | |
|---|---|
| VS∗ = vernis sec ou extrait sec (ce qui reste après évaporation des solvants). | |

### C) Prémontage du lot YED 005B, réalisation du lot YED 005Bx1350

a) Préparation de la suspension de prémontage
• Proportions des excipients mis en oeuvre :

| | |
|---|---|
| EUDRAGIT L 30 D | VS∗ = 30,0 % de la masse d'EUDRAGIT pesée |
| TALC | VS = 10,0 % du VS d'EUDRAGIT |
| TRIETHYL CITRATE | VS = 10,0 % du VS d'EUDRAGIT |
| EAU PURIFIEE | S° = 50,0 % de la masse d'EUDRAGIT pesée |

| | |
|---|---|
| VS∗ = vernis sec ou extrait sec (ce qui reste après évaporation des solvants). | |
| ° S = solvant dans lequel les excipients sont dissous ou dilués. | |

• La suspension est préparée dans un récipient inox dans lequel on introduit l'EAU PURIFIEE,
• Le TRIETHYL CITRATE est incorporé par petites quantités à l'EAU PURIFIEE mise en agitation,
• L'agitation est maintenue jusqu'à homogénéité de la solution (environ 15 minutes),
• L'EUDRAGIT L 30 D est ensuite ajouté,
• Le TALC est incorporé par petites quantités à la suspension,
• L'agitation est maintenue jusqu'à homogénéité du mélange (environ 15 minutes), puis durant tout le prémontage.
b) Prémontage des microgranules de DILTIAZEM
- Les microgranules à enrober (issus du lot YED 005B) sont placés dans une turbine à dragéification,
- Le prémontage des microgranules est effectué par pulvérisation continue de la suspension décrite ci-dessus,
- La masse de microgranules obtenue est tamisée sur une grille d'ouverture de maille de 1,50 mm,
- Les microgranules sont ensuite séchés en turbine en rotation à une température de 40°C pendant 1H puis 7H à température ambiante.

c) Formule du lot YED 005Bx1350 après enrobage protecteur

| Composition duVS∗ | Quantité en % |
|---|---|
| DILTIAZEM | 50,19 % |
| NEUTRES 30 | 14,36 % |
| PVP K 17 | 1,34 % |
| LAURYL SULFATE DE SODIUM | 0,67 % |
| DIETHYL PHTALATE | 0,67 % |
| EUDRAGIT RS 30 D | 18,83 % |
| EUDRAGIT L 30 D | 4,72 % |
| DIBUTYL SEBAÇATE | 0,75 % |
| POLYSORBATE 80 | 0,08 % |
| TALC | 4,91 % |
| TRIETHYL CITRATE | 3,19 % |
| Teneur Théorique | 501,9 mg/g |
| Teneur Trouvée | 521,0 mg/g |

| | |
|---|---|
| VS∗ = vernis sec ou extrait sec (ce qui reste après évaporation des solvants). | |

D) Montage de la seconde couche de Diltiazem sur le lot YED 005Bx1350
a) Préparation de la solution de montage
- Se reporter au paragraphe 5.1 A) a)

b) Montage du DILTIAZEM
- Le montage du principe actif est effectué sur les microgranules issus du prémontage, par pulvérisation discontinue de la suspension décrite ci-dessus en alternance avec des séquences de poudrage du DILTIAZEM, et des séquences de repos,
- La masse de microgranules obtenue est tamisée sur une grille d'ouverture de maille de 1,50 mm,
- Les microgranules sont ensuite séchés dans la turbine en rotation à température ambiante et ce, pendant 8H.

c) Formule du lot YED 005x1350 après montage de la seconde couche de DILTIAZEM

| Composition du VS∗ | Quantité en % |
|---|---|
| DILTIAZEM | 61,40 % |
| NEUTRES 30 | 10,60 % |
| PVP K 17 | 1,91 % |
| LAURYL SULFATE DE SODIUM | 0,95 % |
| DIETHYL PHTALATE | 0,95 % |
| EUDRAGIT RS 30 D | 13,90 % |
| EUDRAGIT L 30 D | 3,48 % |
| DIBUTYL SEBAÇATE | 0,56 % |
| POLYSORBATE 80 | 0,06 % |
| TALC | 3,62 % |
| TRIETHYL CITRATE | 2,57 % |
| Teneur Théorique | 614,0 mg/g |

| | |
|---|---|
| VS∗ = vernis sec ou extrait sec (ce qui reste après évaporation des solvants). | |

### E) Enrobage externe du lot YED 0058x1350

a) Préparation de la suspension d'enrobage
• Proportions des excipients mis en oeuvre :

| | |
|---|---|
| EUDRAGIT RS 30 D | VS∗= 30,0 % de la masse d'EUDRAGIT pesée |
| DIBUTYL SEBACATE | VS = 4,0 % du VS d'EUDRAGIT |
| POLYSORBATE 80 | VS = 0,4 % du VS d'EUDRAGIT |
| TRIETHYL CITRATE | VS = 16,0 % du VS d'EUDRAGIT |
| TALC | VS = 10,0 % du VS d'EUDRAGIT |
| EAU PURIFIEE | S° = 50,0 % de la masse d'EUDRAGIT pesée |

| | |
|---|---|
| VS∗ = vernis sec ou extrait sec (ce qui reste après évaporation des solvants). | |
| ° S = solvant dans lequel les excipients sont dissous ou dilués. | |

• La suspension est préparée dans un récipient inox dans lequel on introduit l'EAU PURIFIEE,
• Le POLYSORBATE 80, le TRIETHYL CITRATE, et le DIBUTYL SEBAÇATE (USP) sont incorporés successivement et par petites quantités à l'EAU PURIFIEE mise en agitation,
• L'agitation est maintenue jusqu'à homogénéité de la solution (environ 15 minutes),
• L'EUDRAGIT RS 30 D est ensuite ajouté,
• L'agitation est maintenue jusqu'à homogénéité du mélange (environ 15 minutes), puis durant tout l'enrobage.
b) Enrobage externe des microgranules de DILTIAZEM
- Les microgranules à enrober sont placés dans une turbine à dragéifi cation,
- L'enrobage des microgranules est effectué par pulvérisation continue de la suspension décrite ci-dessus,
- La masse de microgranules obtenue est tamisée sur une grille d'ouverture de maille de 1,50 mm,
- Les microgranules sont ensuite séchés en turbine en rotation à une température de 40°C pendant 1 h, puis 7h à température ambiante.

c) Formule du lot YED 005Bx1350 après enrobage externe

| Composition duVS∗ | Quantité en % |
|---|---|
| DILTIAZEM | 53,28 % |
| NEUTRES 30 | 9,20 % |
| PVP K 17 | 1,65 % |
| LAURYL SULFATE DE SODIUM | 0,83 % |
| DIETHYL PHTALATE | 0,83 % |
| EUDRAGIT RS 30 D | 21,48 % |
| EUDRAGIT L 30 D | 3,02 % |
| DIBUTYL SEBAÇATE | 0,86 % |
| POLYSORBATE 80 | 0,08 % |
| TALC | 5,03 % |
| TRIETHYL CITRATE | 3,74 % |
| Teneur Théorique | 532,8 mg/g |

| | |
|---|---|
| VS∗ = vernis sec ou extrait sec (ce qui reste après évaporation des solvants). | |

### F) Résultats de dissolution après l'enrobage interne, le montage de la seconde couche de Diltiazem, et l'enrobage externe

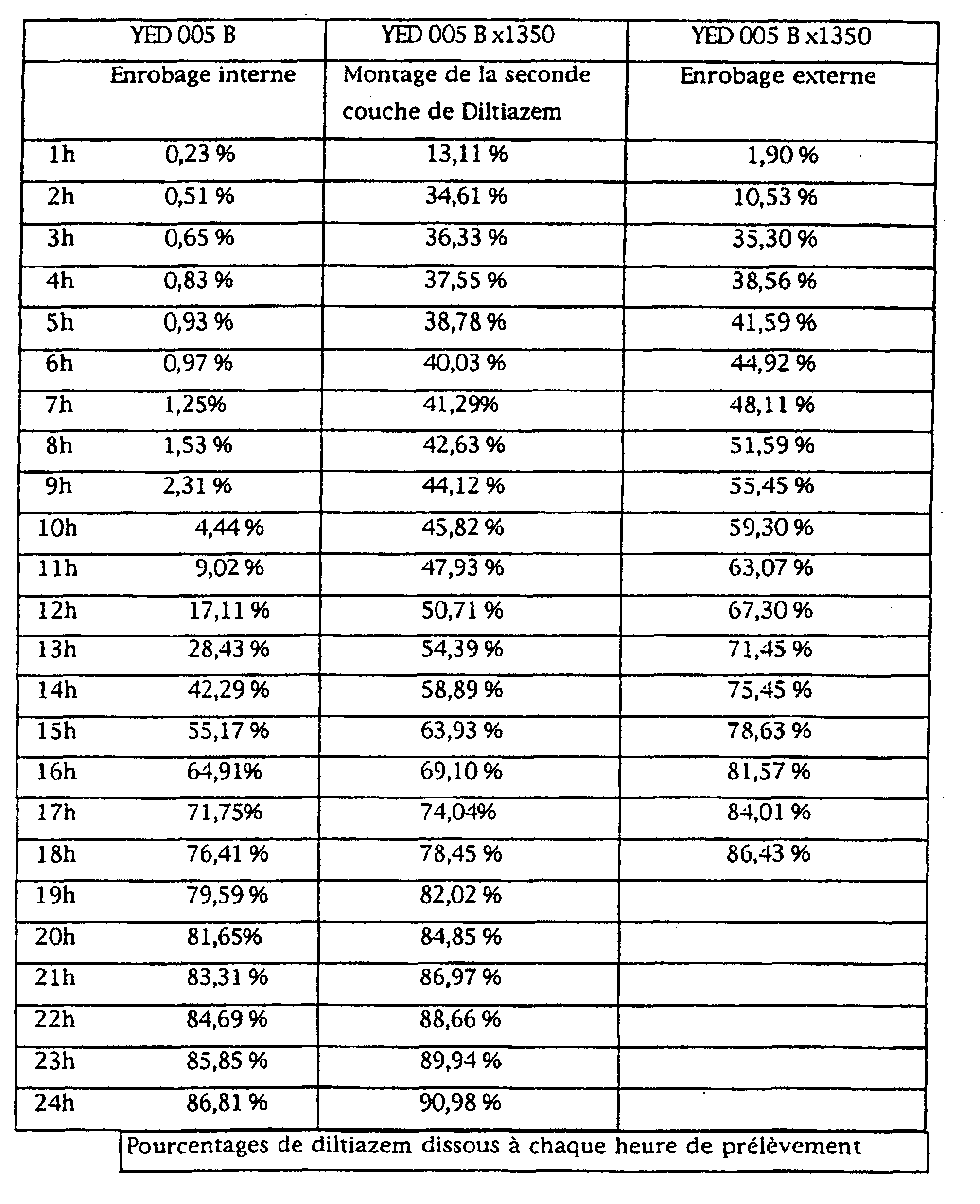

### G) Formules du lot YED 005 B x 1350

- pourcentages donnés en poids pour un total de 100 (hors enrobage) :

| | **YED 005 B x 1350** |
|---|---|
| Montage | |
| Principe actif | 80.99% |
| Support Neutre | 13.98 % |
| Agent liant | 2.52 % |
| Tensio-actif | 1.26 % |
| Plastifiant | 1.26 % |

- pourcentage en poids sec pour un total de 100 % (composition finale) :

| | **YED 005 B x 1350** |
|---|---|
| Support neutre | 9.20 % |

| Montage | |
|---|---|
| Principe actif | 53.28 % |
| Agent liant | 1.65 % |
| Tensio-actif | 0.83 % |
| Plastifiant | 0.83 % |

| Enrobage | |
|---|---|
| Agent d'enrobage | 24.50 % |
| Adjuvant de biodisponibilité | 0.08 % |
| Plastifiant | 4.60 % |
| Lubrifiant | 5.03 % |

### II - Lot YED OO6x1392.2

### A) Montage de la première couche de Diltiazem, réalisation du lot YED 006

a) Préparation de la solution de montage
   - Se reporter au paragraphe 5.1 A) a).
b) Montage du DILTIAZEM sur les grains supports neutres
   - Se reporter au paragraphe 5.1 A) b),
   - La masse obtenue est ensuite lubrifiée avec du TALC.
c) Formule du lot YED 006 après montage de la première couche de Diltiazem
   - Se reporter au paragraphe 5.1 B) a).

### B) Enrobage interne du lot YED 006, réalisation du lot YED006B

a) Préparation de la suspension d'enrobage
- Se reporter au paragraphe 5.2 B) a).

b) Enrobage interne des microgranules de DILTIAZEM (Première étape)
- Les microgranules à enrober (issus du lot YED 006) sont placés dans une turbine perforée,
- L'enrobage des microgranules est effectué à une température de 30°C par pulvérisation continue de la suspension décrite ci-dessus, en alternance avec des séquences de poudrage de TALC (quantité identique à celle mise en suspension),
- La masse de microgranules obtenue est tamisée sur une grille d'ouverture de maille allant de 1,18 à 1,25 mm,
- Les microgranules sont ensuite séchés en turbine en rotation à une température de 30°C pendant 1H, puis 40°C pendant 2H,
- La température est ramenée à 30°C avant de procéder à un second tamisage sur une grille d'ouverture de maille allant de 1,18 à 1,25 mm,
- Les microgranules sont ensuite replacés en turbine en rotation à une température de 30°C et ce, pendant tout l'intervalle de temps entre la phase d'enrobage achevée et la suivante,
- Cette suite d'opération est reproduite jusqu'à obtention de la cinétique désirée,
- A l'issue de l'étape d'enrobage, les microgranules sont tamisés sur une grille d'ouverture de maille de 0,60 mm,
- Ils sont ensuite lubrifiés avec une quantité de TALC équivalente à 0,75 % de la masse enrobée obtenue.

c) Formule du lot YED 006B après enrobage interne

| Composition du VS∗ | Quantité en % |
|---|---|
| DILTIAZEM | 69,18 % |
| NEUTRES 30 | 19,83 % |
| PVP K 17 | 2,02 % |
| LAURYL SULFATE DE SODIUM | 1,01 % |
| DIETHYL PHTALATE | 1,01 % |
| EUDRAGIT RS 30 D | 4,66 % |
| DIBUTYL SEBAÇATE | 0,19 % |
| POLYSORBATE 80 | 0,02 % |
| TALC | 1,35 % |
| TRIETHYL CITRATE | 0,75 % |
| Teneur Théorique | 691,8 mg/g |

| | |
|---|---|
| VS∗ = vernis sec ou extrait sec (ce qui reste après évaporation des solvants). | |

### C) Enrobage interne du lot YED 006B, réalisation du lot YED006Bx1392

a) Préparation de la suspension d'enrobage
- Se reporter au paragraphe 5.1 B) a).

b) Enrobage interne des microgranules de DILTIAZEM (Deuxième étape)
- Les microgranules à enrober (issus du lot YED 006B) sont placés dans une turbine perforée,
- L'enrobage des microgranules est effectué par pulvérisation continue de la suspension décrite ci-dessus,
- La masse de microgranules obtenue est tamisée sur une grille d'ouverture de maille allant de 1,18 à 1,25 mm,
- Les microgranules sont ensuite séchés en turbine en rotation à une température de 30°C pendant 1H, puis 40°C pendant 2H,
- La température est ramenée à 30°C avant de procéder à un second tamisage sur une grille d'ouverture de maille allant de 1,18 à 1,25 mm,
- Les microgranules sont ensuite replacés en turbine en rotation à une température de 30°C et ce, pendant tout l'intervalle de temps entre la phase d'enrobage achevée et la suivante,
- Cette suite d'opération est reproduite jusqu'à obtention de la cinétique désirée,
- A l'issue de l'étape d'enrobage, les microgranules sont tamisés sur une grille d'ouverture de maille de 0,60 mm.

c) Formule du lot YED 0068x1392 après enrobage interne

| Composition du VS∗ | Quantité % |
|---|---|
| DILTIAZEM | 52,29 % |
| NEUTRES 30 | 14,99 % |
| PVP K 17 | 1,53 % |
| LAURYL SULFATE DE SODIUM | 0,76 % |
| DIETHYL PHTALATE | 0,76 % |
| EUDRAGIT RS 30 D | 20,91 % |
| DIBUTYL SEBAÇATE | 0,84 % |
| POLYSORBATE 80 | 0,08 % |
| TALC | 4,49 % |
| TRIETHYL CITRATE | 3,35 % |
| Teneur Théorique | 522,9 mg/g |

| | |
|---|---|
| VS∗ = vernis sec ou extrait sec (ce qui reste après évaporation des solvants). | |

### D) Prémontage du lot YED 006Bx1392, réalisation du lot YED006Bx1392.2

a) Préparation de la suspension de prémontage
• Proportions des excipients mis en oeuvre :

| | |
|---|---|
| EUDRAGIT L 30 D | VS∗= 30,0 % de la masse d'EUDRAGIT pesée |
| TRIETHYL CITRATE | VS = 10,0 % du VS d'EUDRAGIT |
| EAU PURIFIEE | S° = 20,0 % de la masse d'EUDRAGIT pesée |

| | |
|---|---|
| VS∗ = vernis sec ou extrait sec (ce qui reste après évaporation des solvants). | |
| ° S = solvant dans lequel les excipients sont dissous ou dilués. | |

• La suspension est préparée dans un récipient inox dans lequel on introduit l'EAU PURIFIEE,
• Le TRIETHYL CITRATE est incorporé par petites quantités à l'EAU PURIFIEE mise en agitation,
• L'agitation est maintenue jusqu'à homogénéité de la solution (environ 15 minutes),
• L'EUDRAGIT L 30 D est ensuite ajouté,
• L'agitation est maintenue jusqu'à homogénéité du mélange (environ 15 minutes), puis durant tout le prémontage.
b) Prémontage des microgranules de DILTIAZEM
- Les microgranules à enrober sont placés dans une turbine perforée,
- Le prémontage des microgranules est effectué à une température de 30°C par pulvérisation con tinue de la suspension décrite ci-dessus,
- La masse de microgranules obtenue est tamisée sur une grille d'ouverture de maille de 1,40 mm,
- Les microgranules sont ensuite séchés en turbine en rotation à une température de 35°C pendant 10H.

c ) Formule du lot YED 00613x1392.2 après prémontage

| Composition du VS∗ | Quantité en % |
|---|---|
| DILTIAZEM | 50,19 % |
| NEUTRES 30 | 14,36 % |
| PVP K 17 | 1,34 % |
| LAURYL SULFATE DE SODIUM | 0,67 % |
| DIETHYL PHTALATE | 0,67 % |
| EUDRAGIT RS 30 D | 18,83 % |
| EUDRAGIT L 30 D | 4,72 % |
| DIBUTYL SEBAÇATE | 0,75 % |
| POLYSORBATE 80 | 0,08 % |
| TALC | 4,91 % |
| TRIETHYL CITRATE | 3,19 % |
| Teneur Théorique | 501,9 mg/g |

| | |
|---|---|
| VS∗ = vernis sec ou extrait sec (ce qui reste après évaporation des solvants). | |

### E) Montage de la seconde couche de DILTIAZEM sur le lot YED 006Bx1392.2

a) Préparation de la solution de montage
- Se reporter au paragraphe 5.2 A) a).

b) Montage du DILTIAZEM
- Le montage du principe actif est effectué sur les microgranules issus de l'enrobage protecteur du lot YED006Bx1392.2, par pulvérisation discontinue de la suspension décrite ci-dessus en alternance avec des séquences de poudrage du DILTIAZEM, et des séquences de repos,
- La masse de microgranules obtenue est tamisée sur une grille d'ouverture de maille de 1,50 mm,
- Les microgranules sont ensuite séchés dans la turbine en rotation à température ambiante et ce, pendant 8H.

c) Formule du lot YED 0068x13912.2 après montage de la seconde couche de DILTIAZEM

| Composition du VS∗ | Quantité en % |
|---|---|
| DILTIAZEM | 58,75 % |
| NEUTRES 30 | 10,11 % |
| PVP K 17 | 1,85 % |
| LAURYL SULFATE DE SODIUM | 0,93 % |
| DIETHYL PHTALATE | 0,92 % |
| EUDRAGIT RS 30 D | 14,11 % |
| EUDRAGIT L 30 D | 6,75 % |
| DIBUTYL SEBAÇATE | 0,56 % |
| POLYSORBATE 80 | 0,06 % |
| TALC | 3,03 % |
| TRIETHYL CITRATE | 2,93 % |
| Teneur Théorique | 587,5 mg/g |

| | |
|---|---|
| VS∗ = vernis sec ou extrait sec (ce qui reste après évaporation des solvants). | |

### F) Enrobage externe du lot YED 006Bx1392.2

a) Préparation de la suspension d'enrobage
- Se reporter au paragraphe 5.2 B) a).

b) Enrobage externe des microgranules de DILTIAZEM
- Les microgranules à enrober sont placés dans une turbine perforée,
- L'enrobage des microgranules est effectué par pulvérisation continue de la suspension décrite ci-dessus,
- La masse de microgranules obtenue est tamisée sur une grille d'ouverture de maille allant de 1,18 à 1,25 mm,
- Les microgranules sont ensuite séchés en turbine en rotation à une température de 30°C pendant 1H, puis 40°C pendant 2H,
- La température est ramenée à 30°C avant de procéder à un second tamisage sur une grille d'ouverture de maille allant de 1,18 à 1,25 mm,
- Les microgranules sont ensuite replacés en turbine en rotation à une température de 30°C et ce, pendant tout l'intervalle de temps entre la phase d'enrobage achevée et la suivante,
- Cette suite d'opération est reproduite jusqu'à obtention de la cinétique désirée,
- A l'issue de l'étape d'enrobage, les microgranules sont tamisés sur une grille d'ouverture de maille de 0,60 mm,
- Ils sont ensuite lubrifiés avec une quantité de TALC équivalente à 1,5 % de la masse enrobée obtenue.

c) Formule du lot YED 006Bx1392.2 après enrobage externe

| Composition duVS∗ | Quantité en % |
|---|---|
| DILTIAZEM | 49,71 % |
| NEUTRES 30 | 8,56 % |
| PVP K 17 | 1,57 % |
| LAURYL SULFATE DE SODIUM | 0,78 % |
| DIETHYL PHTALATE | 0,78 % |
| EUDRAGIT RS 30 D | 21,89 % |
| EUDRAGIT L 30 D | 5,71 % |
| DIBUTYL SEBAÇATE | 0,88 % |
| POLYSORBATE 80 | 0,09 % |
| TALC | 5,97 % |
| TRIETHYL CITRATE | 4,08 % |
| Teneur Théorique | 497,1 mg/g |

| | |
|---|---|
| VS∗ = vernis sec ou extrait sec (ce qui reste après évaporation des solvants). | |

### G) Résultats de dissolution après l'enrobage interne et l'enrobage externe (dans deux milieux)

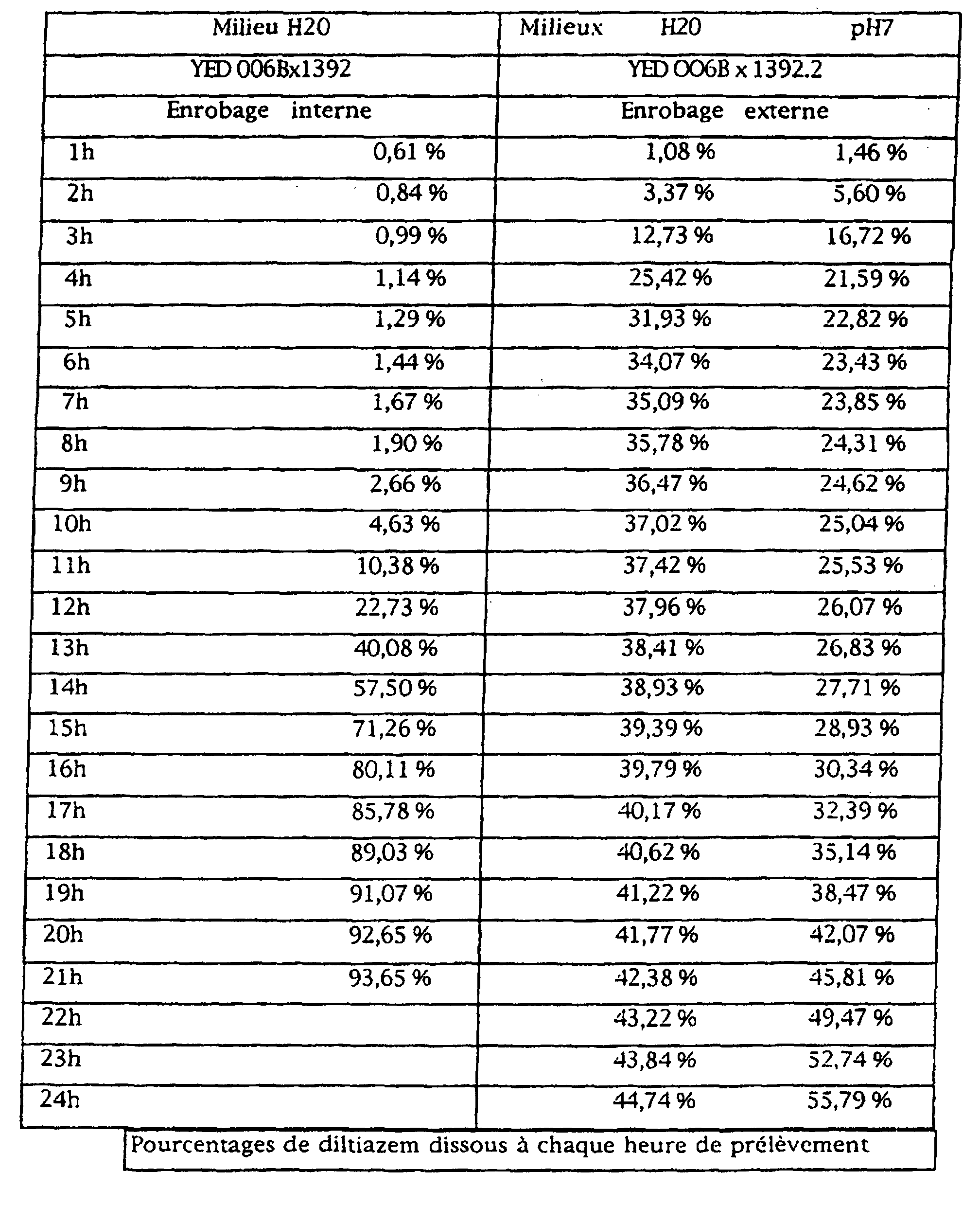

### H) Formules du lot YED 006 x 1392.2

- pourcentages donnés en poids pour un total de 100 (hors enrobage) :

| | **YED 006 x 1392.2** |
|---|---|
| Montage | |
| Principe actif | 80.96 % |
| Support Neutre | 13.93 % |
| Agent liant | 2.55 % |
| Tensio-actif | 1.28 % |
| Plastifiant | 1.27 % |

- pourcentage en poids sec pour un total de 100 % (composition finale) :

| | **YED 006 x 1392.2** |
|---|---|
| Support neutre | 8.56 % |

| Montage | |
|---|---|
| Principe actif | 49.71 % |
| Agent liant | 1.57 % |
| Tensio-actif | 0.78 % |
| Plastifiant | 0.78 % |

| Enrobage | |
|---|---|
| Agent d'enrobage | 27.59 % |
| Adjuvant de biodisponibilité | 0.09 % |
| Plastifiant | 4.96 % |
| Lubrifiant | 5.97 % |

### EXEMPLE 6 - Préparation de microgranules mono-couche

### A) Montage du Diltiazem, réalisation du lot YED 001

a) Préparation de la solution de montage
• Proportions des excipients mis en oeuvre :

| | | |
|---|---|---|
| PVP K 17 | 50% du VS* total | VS = 15 % du poids de la solution |
| LAURYL SULFATE DE SODIUM | 25% du VS total | |
| DIETHYL PHTALATE | 25% du VS total | |
| EAU PURIFIEE | 50% du S° total | S = 85 % du poids de la solution |
| ALCOOL ETHYLIQUE 95 % | 50% du S total | |

| | | |
|---|---|---|
| * VS = vernis sec ou extrait sec (ce qui reste après évaporation des solvants). | | |
| ° S = solvants dans lesquels les excipients sont dissous ou dilués. | | |

• La solution est préparée dans un récipient inox,
• L'ALCOOL ETHYLIQUE 95 % puis l'EAU PURIFIEE sont versés dans le récipient puis mis en agitation,
• Le PVP K 17 puis le LAURYL SULFATE sont incorporés successivement et par petites qantités,
• L'agitation est maintenue jusqu'à complète dissolution du PVP K 17 et du LAURYL SULFATE,
• Le DIETHYL PHTALATE est ensuite ajouté et, l'agitation est maintenue jusqu'à homogénéité de la solution.
b) Montage du DILTIAZEM sur les grains supports neutres
- Des grains supports NEUTRES 30 sont placés dans une turbine à dragéification en rotation,
- Le montage du principe actif est effectué sur les NEUTRES 30, par pulvérisation discontinue de la solution décrite ci-dessus en alternance avec des séquences de poudrage du DILTIAZEM, et des séquences de repos,
- La masse de microgranules obtenue est tamisée sur une grille d'ouverture de maille allant de 0,71 à 1,00 mm,
- Les microgranules sont ensuite séchés dans la turbine en rotation à température ambiante et pendant 3 à 8H.

c) Formule du lot YED 001 après montage

| Composition du VS* | Quantité en % |
|---|---|
| DILTIAZEM | 73,05 % |
| NEUTRES 30 | 22,55 % |
| PVP K 17 | 2,20 % |
| LAURYL SULFATE DE SODIUM | 1,10 % |
| DIETHYL PHTALATE | 1,10 % |
| Teneur théorique | 730,50 mg/g |
| Teneur obtenue | 735,75 mg/g |

| | |
|---|---|
| * VS = vernis sec ou extrait sec (ce qui reste après évaporation des solvants). | |

### B) Enrobage du lot YED 001, réalisation des lots YED 001A1 et YED 001B1

Après séparation de la masse de microgranules obtenus au montage (proportions : 45%/55% m/m), les deux masses sont enrobées séparément avec des quantités d'excipients différentes.
a) Préparation de la suspension d'enrobage
• Proportions des excipients mis en oeuvre :

| | |
|---|---|
| EUDRAGIT RS 30 D | VS*= 30,0 % de la masse d'EUDRAGIT pesée |
| DIBUTYL SEBACATE | VS = 4,0 % du VS d'EUDRAGIT |
| POLYSORBATE 80 | VS = 0,4 % du VS d'EUDRAGIT |
| TRIETHYL CITRATE | VS = 16,0 % du VS d'EUDRAGIT |
| TALC | VS = 10,0 % du VS d'EUDRAGIT |
| EAU PURIFIEE | S°= 50,0 % de la masse d'EUDRAGIT pesée |

| | |
|---|---|
| * VS = vernis sec ou extrait sec (ce qui reste après évaporation des solvants). | |
| ° S = solvant dans lequel les excipients sont dissous ou dilués. | |

• La suspension est préparée dans un récipient inox dans lequel on introduit l'EAU PURIFIEE,
• Le POLYSORBATE 80, le TRIETHYL CITRATE, et le DIBUTYL SEBACATE (USP) sont incorporés successivement et par petites quantités à l'EAU PURIFIEE mise en agitation,
• L'agitation est maintenue jusqu'à homogénéité de la suspension (environ 15 minutes),
• L'EUDRAGIT RS 30 D et le talc sont ensuite ajoutés,
• L'agitation est maintenue jusqu'à homogénéité du mélange (environ 15 minutes), puis durant tout l'enrobage.
b) Enrobage des microgranules de DILTIAZEM
- Les microgranules à enrober (issus du lot YED 001) sont placés dans une turbine à dragéification,
- L'enrobage des microgranules est effectué à une température de 30°C par pulvérisation continue de la suspension décrite ci-dessus, en alternance avec des séquences de poudrage de TALC (VS=20% du VS d'EUDRAGIT),
- La masse de microgranules obtenue est tamisée sur une grille d'ouverture de maille allant de 1,00 à 1,12 mm,
- Les microgranules sont ensuite séchés en turbine en rotation à température ambiante et ce, pendant tout l'intervalle de temps entre la phase d'enrobage achevée et la suivante,
- Cette suite d'opération est reproduite jusqu'à obtention de la cinétique désirée pour les lots A1 et B1.

c) Formule des lots YED 001A1, YED 001B1 après enrobage, mélange YED 001M3
• A l'issue des étapes d'enrobage, une fraction des granules issus du lot YED 001A1 et une fraction des granules issus du lot YED 001B1 sont mélangés et lubrifiés avec du TALC (VS de TALC = 0,50 % de la masse totale mélangée),
• Le mélange est effectué de telle sorte que la quantité de principe actif amenée par le lot YED 001A1 soit égale à 40 % de la quantité totale de principe actif dans le mélange. Les 60 % restants sont amenés par le lot YED 001B1.

| Lot | YET OO1A1 | YED 001B1 | YED 001M3 |
|---|---|---|---|
| Composition du VS* | Quantité en % | | |
| DILTIAZEM | 61,5 % | 45,1 % | 50,4 % |
| NEUTRES 30 | 19,0 % | 13,9 % | 15,5 % |
| PVP K 17 | 1,9 % | 1,4 % | 1,5 % |
| LAURYL SULFATE DE SODIUM | 0,9 % | 0,7 % | 0,8 % |
| DIETHYL PHTALATE | 0,9 % | 0,7 % | 0,8 % |
| EUDRAGIT RS 30 D | 11,3 % | 27,2 % | 21,7 % |
| DIBUTYL SEBACATE | 0,4 % | 1,1 % | 0,9 % |
| POLYSORBATE 80 | 0,1 % | 0,1 % | 0,1 % |
| TALC | 2,3 % | 5,5 % | 4,9 % |
| TRIETHYL CITRATE | 1,8 % | 4,4 % | 3,5 % |
| Teneur Théorique | 615mg/g | 451mg/g | 504mg/g |
| Teneur Trouvé | 602mg/g | 453mg/g | 497mg/g |

| | | | |
|---|---|---|---|
| * VS = vernis sec ou extrait sec (ce qui reste après évaporation des solvants). | | | |

### C) Résultats de dissolution des lots YED 001A1, YED 001B1, YED 001M3, 8175 (gélules du lot YED 001 M3 dosées à 300 mg)

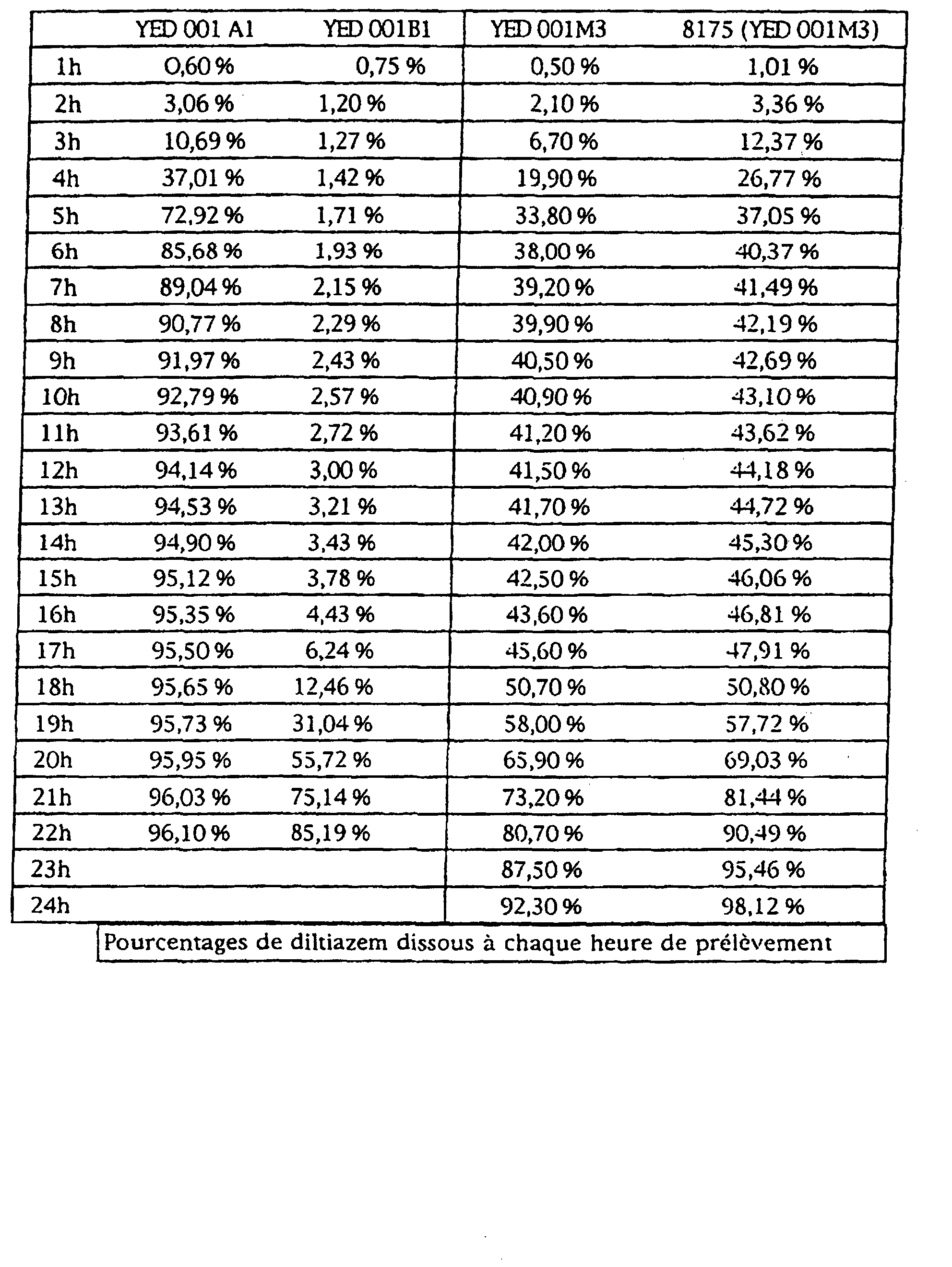

### D) Formules du lot YED 001 M3

- pourcentages donnés en poids pour un total de 100 (hors enrobage) :

| | **YED 001 M3** |
|---|---|
| Montage | |
| Principe actif | 73.05 % |
| Support Neutre | 22.55 % |
| Agent liant | 2.20 % |
| Tensio-actif | 1.10 % |
| Plastifiant | 1.10 % |

- pourcentage en poids sec pour un total de 100 % (composition finale) :

| | **YED 001 M3** |
|---|---|
| Support neutre | 15.55 % |

| Montage | |
|---|---|
| Principe actif | 50.37 % |
| Agent liant | 1.52 % |
| Tensio-actif | 0.76 % |
| Plastifiant | 0.76 % |

| Enrobage | |
|---|---|
| Agent d'enrobage | 21.72 % |
| Adjuvant de biodisponibilité | 0.09 % |
| Plastifiant | 4.37 % |
| Lubrifiant | 4.86 % |

### EXEMPLE 7 - Préparation de microgranules mono-couche

### A) Montage du Diltiazem, réalisation des lots YED 004 et YED 005

a) Préparation de la solution de montage
• Proportions des excipients mis en oeuvre :

| | | |
|---|---|---|
| PVP K 17 | 50 % du VS* total | |
| LAURYL SULFATE DE SODIUM | 25 % du VS total | VS = 20 % du poids de la solution |
| DIETHYL PHTALATE | 25 % du VS total | |
| EAU PURIFIEE | 50 % duS° total | S = 20 % du poids de la solution |
| ALCOOL ETHYLIQUE 95 % | 50 % du S total | |

| | | |
|---|---|---|
| * VS = vernis sec ou extrait sec (ce qui reste après évaporation des solvants). | | |
| ° S = solvant dans lequel les excipients sont dissous ou dilués. | | |

• La solution est préparée dans un récipient inox,
• L'ALCOOL ETHYLIQUE 95 % puis l'EAU PURIFIEE sont versés dans le récipient puis mis en agitation,
• Le PVP K 17 puis le LAURYL SULFATE sont incorporés successivement et par petites quantités,
• L'agitation est maintenue jusqu'à complète dissolution du PVP K 17 et du LAURYL SULFATE,
• Le DIETHYL PHTALATE est ensuite ajouté et, l'agitation est maintenue jusqu'à homogénéité de la solution.
b) Montage du DILTIAZEM sur les grains supports neutres
- Des grains supports NEUTRES 30 sont placés dans une turbine à dragéification en rotation,
- Le montage du principe actif est effectué sur les NEUTRES 30, par pulvérisation discontinue de la suspension décrite ci-dessus en alternance avec des séquences de poudrage du DILTIAZEM, et des séquences de repos,
- La masse de microgranules obtenue est tamisée sur une grille d'ouverture de maille allant de 0,85 à 1,18 mm,
- Les microgranules sont ensuite séchés dans la turbine en rotation
   * YED 004 : à 35°C et pendant 12 à 14H,
   * YED 005:. A température ambiante et pendant 2H30 entre deux phases consécutives.
      . A 35°C et pendant 4 à 6H pour la dernière phase de montage de chaque journée.
      . A l'issue du séchage, la turbine est laissée en rotation 4H sans apport de chaleur.

c) Formule des lots YED 004 et YED 005 après montage

| LOT | YED 004 | YED 005 |
|---|---|---|
| Composition du VS* | Quantité en % | Quantité en % |
| DILTIAZEM | 73,94 | 74,65 |
| NEUTRES 30 | 21,20 | 21,37 |
| PVP K 17 | 2,43 | 1,99 |
| LAURYL SULFATE DE SODIUM | 1,21 | 1,00 |
| DIETHYL PHTALATE | 1,21 | 1,00 |
| TENEUR THORIQUE | 739,4 mg/g | 746,5 mg/g |
| TENEUR OBTENUE | 727,8 mg/g | 711,0 mg/g |

| | | |
|---|---|---|
| * VS = vernis sec ou extrait sec (ce qui reste après évaporation des solvants). | | |

B) Enrobage des lots YED 004 et YED 005, réalisation des lots YED 004A et YED 005B
Après séparation des masses de microgranules obtenues au montage (proportions : 40 %/60 % m/m), deux des masses YED 004A (40 % de YED 004) et YED 005B (60 % de YED 005) sont enrobées séparément avec des quantités d'excipients différentes.
a) Préparation de la suspension d'enrobage
• Proportions des excipients mis en oeuvre :

| | |
|---|---|
| EUDRAGIT RS 30 D | VS*= 30,0 % de la masse d'EUDRAGIT pesée |
| DIBUTYL SEBACATE | VS = 4,0 % du VS d'EUDRAGIT |
| POLYSORBATE 80 | VS = 0.4 % du VS d'EUDRAGIT |
| TALC | VS = 10,0 % du VS d'EUDRAGIT |
| TRIETHYL CITRATE | VS = 16,0 % du VS d'EUDRAGIT |
| EAU PURIFIEE | S° = 50,0 % de la masse d'EUDRAGIT pesée |

| | |
|---|---|
| * VS = vernis sec ou extrait sec (ce qui reste après évaporation des solvants). | |
| ° S = solvant dans lequel les excipients sont dissous ou dilués. | |

• La suspension est préparée dans un récipient inox dans lequel on introduit l'EAU PURIFIEE,
• Le POLYSORBATE 80, le TRIETHYL CITRATE, et le DIBUTYL SEBACATE (USP) sont incorporés successivement et par petites quantités à l'EAU PURIFIEE mise en agitation,
• L'agitation est maintenue jusqu'à homogénéité de la suspension (environ 15 minutes),
• L'EUDRAGIT RS 30 D est ensuite ajouté,
• Le TALC est incorporé par petites quantités à la suspension,
• L'agitation est maintenue jusqu'à homogénéité du mélange (environ 15 minutes), puis durant tout l'enrobage,
b) Enrobage des microgranules de DILTIAZEM
- Les microgranules à enrober (issus des lots YED 004 et YED 005) sont placés dans une turbine perforée,
- L'enrobage des microgranules est effectué à une température de 30°C par pulvérisation continue de la suspension décrite ci-dessus,
- La masse de microgranules obtenue est tamisée sur une grille d'ouverture de maille allant de 1,18 à 1,25 mm,
- Les microgranules sont ensuite séchés en turbine en rotation à une température de 30°C pendant 1H puis 40°C pendant 2H,
- La température est ramenée à 30°C avant de procéder à un second tamisage sur une grille d'ouverture de maille allant de 1,18 à 1,25 mm,
- Les microgranules sont ensuite replacés en turbine en rotation à une température de 30°C et ce, pendant tout l'intervalle de temps entre la phase d'enrobage achevée et la suivante,
- Cette suite d'opération est reproduite jusqu'à obtention de la cinétique désirée,
- A l'issue de l'étape d'enrobage, les microgranules sont tamisés sur une grille d'ouverture de maille de 0,60 mm,
- Ils sont ensuite lubrifiés avec une quantité de TALC équivalente à 0,75 % de la masse enrobée obtenue.

c) Formule des lots YED 004A et YED 005B après enrobage, mélange YED 005M
• Une fraction des granules issus du lot YED 004A et une fraction des granules issus du lot YED 005 B sont mélangés,
• Le mélange est effectué de telle sorte que la quantité de principe actif amenée par le lot YED 004A soit égale à 40 % de la quantité totale de principe actif dans le mélange. Les 60 % restants sont amenés par le lot YED 005B.

| LOT | YED 004A | YED 005B | YED 005M |
|---|---|---|---|
| Composition du VS* | Quantité en % | Quantité en % | Quantité en % |
| DILTIAZEM | 65,37 | 53,20 | 57,43 |
| NEUTRES 30 | 18,75 | 15,23 | 16,45 |
| PVP K 17 | 2,15 | 1,42 | 1,68 |
| LAURYL SULFATE DE SODIUM | 1,07 | 0,71 | 0,84 |
| DIETHYL PHTALATE | 1,07 | 0,71 | 0,84 |
| EUDRAGIT PS 30 D | 7,74 | 19,96 | 15,67 |
| DIBUTYL SEBACATE | 0,31 | 0,80 | 0,63 |
| POLYSORBATE 80 | 0,04 | 0,08 | 0,07 |
| TALC | 2,27 | 4,70 | 3,91 |
| TRIETHYL CITRATE | 1,24 | 3,19 | 2,51 |
| TENEUR THEORIQUE | 654 mg/g | 532 mg/g | 574 mg/g |
| TENEUR TROUVEE | 656 mg/g | 537 mg/g | 540 mg/g |

| | | | |
|---|---|---|---|
| * VS = vernis sec ou extrait sec (ce qui reste après évaporation des solvants). | | | |

### C) Résultats de dissolution des lots YED 004A, YED 005B, YED 005M, 8394 (Gélules du lot YED 005M dosées à 300 mg)

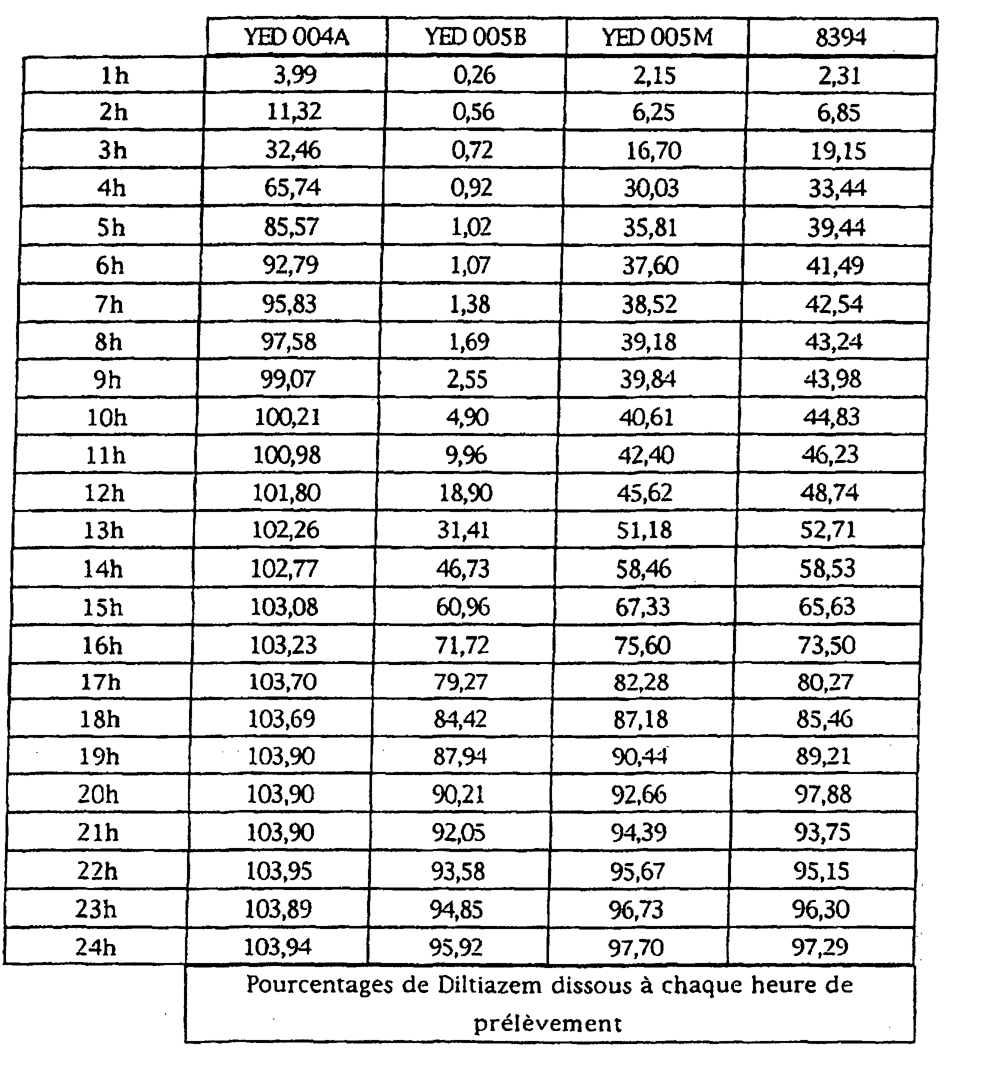

### D) Formules du lot YED 005 M

- pourcentages donnés en poids pour un total de 100 (hors enrobage) :

| | **YED 005 M** |
|---|---|
| Montage | |
| Principe actif | 74.37 % |
| Support Neutre | 21.30 % |
| Agent liant | 2.17 % |
| Tensio-actif | 1.08 % |
| Plastifiant | 1.08 % |

- pourcentage en poids sec pour un total de 100 % (composition finale) :

| | **YED 005 M** |
|---|---|
| Support neutre | 16.45 % |

| Montage | |
|---|---|
| Principe actif | 57.43 % |
| Agent liant | 1.68 % |
| Tensio-actif | 0.84 % |
| Plastifiant | 0.84 % |

| Enrobage | |
|---|---|
| Agent d'enrobage | 15.67 % |
| Adjuvant de biodisponibilité | 0.07 % |
| Plastifiant | 3.13 % |
| Lubrifiant | 3.91 % |

### EXEMPLE 8 - Préparation de microgranules double couche

### A) Montage de la première couche de Diltiazem, réalisation du lot YED 005

- Se reporter au paragraphe A) de l'exemple 5.

### B) Enrobage interne du lot YED 005, réalisation du lot YED 005x1406

a) Préparation de la suspension d'enrobage
- Se reporter au paragraphe B) a) de l'exemple 5.

b) Enrobage interne des microgranules de DILTIAZEM
- Les microgranules à enrober sont placés dans une turbine à dragéification,
- L'enrobage des microgranules est effectué par pulvérisation continue de la suspension décrite ci-dessus,
- La masse de microgranules obtenue est tamisée sur une grille d'ouverture de maille de 1,50mm,
- Les microgranules sont ensuite séchés en turbine en rotation à une température de 40°C pendant 2H.

c) Formule du lot YED 005x1406 après enrobage interne

| Composition des VS* | Quantité en % |
|---|---|
| DILTIAZEM | 58,12 |
| NEUTRES 30 | 16,63 |
| PVP K 17 | 1,55 |
| LAURYL SULFATE DE SODIUM | 0,78 |
| DIETHYL PHTALATE | 0,78 |
| EUDRAGIT RS 30 D | 15,61 |
| DIBUTYL SEBACATE | 0,62 |
| POLYSORBATE 80 | 0,06 |
| TALC | 3,35 |
| TRIETHYL CITRATE | 2,50 |
| TENEUR THEORIQUE | 581,2 mg/g |

| | |
|---|---|
| * VS = vernis sec ou extrait sec (ce qui reste après évaporation des solvants). | |

### C) Prémontage du lot YED 005x1406

a) Préparation de la suspension de prémontage
- Se reporter au paragraphe II D) a) de l'exemple 5.2.

b) Prémontage des microgranules de DILTIAZEM
- Les microgranules à prémonter sont placés dans une turbine à dragéification,
- Le prémontage des microgranules est effectué à une température de 30°C par pulvérisation continue de la suspension décrite ci-dessus,
- La masse de microgranules obtenue est tamisée sur une grille d'ouverture de maille de 1,50 mm,
- Les microgranules sont ensuite séchés en turbine en rotation à une température de 35°C pendant 2H.

c) Formule du lot YED 005x1406 après prémontage

| Composition duVS* | Quantité en % |
|---|---|
| DILTIAZEM | 52,36 |
| NEUTRES 30 | 14,98 |
| PVP K 17 | 1,40 |
| LAURYL SULFATE DE SODIUM | 0,70 |
| DIETHYL PHTALATE | 0,70 |
| EUDRAGIT RS 30 D | 14,06 |
| EUDRAGIT L 30 D | 9,01 |
| DIBUTYL SEBACATE | 0,56 |
| POLYSORBATE 80 | 0,06 |
| TALC | 3,02 |
| TRIETHYL CITRATE | 3,16 |
| TENEUR THEORIQUE | 523,6 mg/g |

| | |
|---|---|
| * VS = vernis sec ou extrait sec (ce qui reste après évaporation des solvants). | |

### D) Montage de la seconde couche de DILTIAZEM sur le lot YED 005x1406

a) Préparation de la solution de montage
- Se reporter au paragraphe A) a) de l'exemple 5.

b) Montage du DILTIAZEM
- Le montage du principe actif est effectué sur les microgranules issus de l'enrobage protecteur du lot YED 005x1406, par pulvérisation discontinue de la suspension décrite ci-dessus en alternance avec des séquences de poudrage du DILTIAZEM, et des séquences de repos,
- La masse de microgranules obtenue est tamisée sur une grille d'ouverture de maille de 1,60 à 1,80 mm,
- Les microgranules sont ensuite séchés dans la turbine en rotation à température ambiante et ce, pendant 12H.

c) Formule du lot YED 005x1406 après montage de la seconde couche de DILTIAZEM

| Composition duVS* | Quantité en % |
|---|---|
| DILTIAZEM | 62.58 |
| NEUTRES 30 | 11.08 |
| PVP K 17 | 2.13 |
| LAURYL SULFATE DE SODIUM | 1.06 |
| DIETHYL PHTALATE | 1.06 |
| EUDRAGIT RS 30 D | 10.40 |
| EUDRAGIT L 30 D | 6.66 |
| DIBUTYL SEBACATE | 0.41 |
| POLYSORBATE 80 | 0.04 |
| TALC | 2.23 |
| TRIETHYL CITRATE | 2.33 |
| TENEUR THEORIQUE | 625.8 mg/g |

| | |
|---|---|
| * VS = vernis sec ou extrait sec (ce qui reste après évaporation des solvants). | |

### E) Enrobage externe du lot YED 005x1406

a) Préparation de la suspension d'enrobage
- Se reporter au paragraphe B) a) de l'exemple 5.

b) Enrobage externe des microgranules de DILTIAZEM
- Les microgranules à enrober sont placés dans une turbine à dragéification,
- L'enrobage des microgranules est effectué par pulvérisation continue de la suspension décrite ci-dessus,
- La masse de microgranules obtenue est tamisée sur une grille d'ouverture de maille de 1,50 mm,
- Les microgranules sont ensuite séchés en turbine en rotation à une température de 40°C pendant 1H, puis 7H à température ambiante.

c) Formule du lot YED 005x1406 après enrobage externe

| Composition du VS* | Quantité en % |
|---|---|
| DILTIAZEM | 55,46 |
| NEUTRES 30 | 9,82 |
| PVP K 17 | 1,88 |
| LAURYL SULFATE DE SODIUM | 0,94 |
| DIETHYL PHTALATE | 0,94 |
| EUDRAGIT RS 30 D | 16,30 |
| EUDRAGIT L 30 D | 5,91 |
| DIBUTYL SEBACATE | 0,65 |
| POLYSORBATE 80 | 0,07 |
| TALC | 4,82 |
| TRIETHYL CITRATE | 3,20 |
| TENEUR THEORIQUE | 554,6 mg/g |

| | |
|---|---|
| * VS = verni sec ou extrait sec (ce qui reste après évaporation des solvants). | |

### F) Résultats de dissolution après l'enrobage interne et l'enrobage externe (dans 2 milieux)

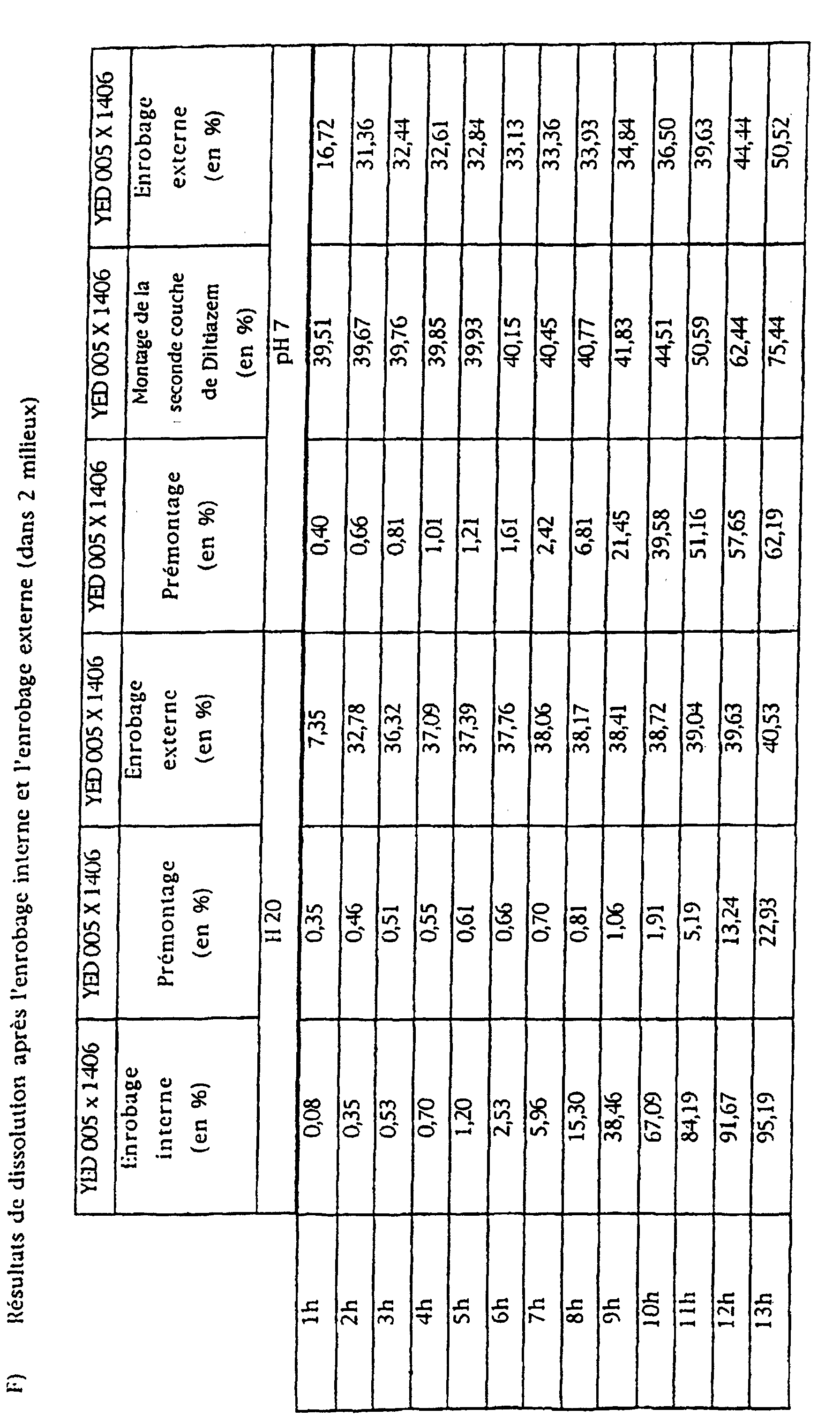

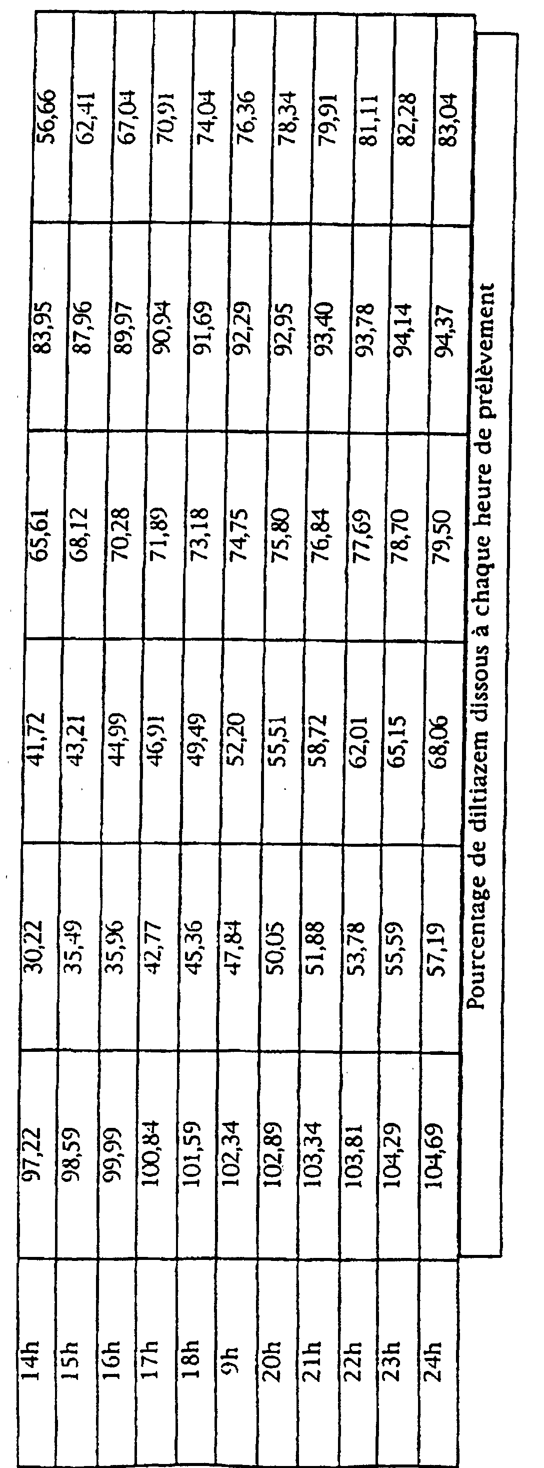

### G) Formules du lot YED 005 x 1406

- pourcentages donnés en poids pour un total de 100 (hors enrobage) :

| | **YED 005 x 1406** |
|---|---|
| Montage | |
| Principe actif | 80.32 % |
| Support Neutre | 14.22 % |
| Agent liant | 2.73 % |
| Tensio-actif | 1.36 % |
| Plastifiant | 1.36 % |

- pourcentage en poids sec pour un total de 100 % (composition finale) :

| | **YED 005 x 1406** |
|---|---|
| Support neutre | 9.82 % |

| Montage | |
|---|---|
| Principe actif | 55.46 % |
| Agent liant | 1.88 % |
| Tensio-actif | 0.94 % |
| Plastifiant | 0.94 % |

| Enrobage | |
|---|---|
| Agent d'enrobage | 22.21 % |
| Adjuvant de biodisponibilité | 0.07 % |
| Plastifiant | 3.85 % |
| Lubrifiant | 4.82 % |

## Revendications

1. Microgranules à libération prolongée (LP) contenant du diltiazem et exempts d'acide organique hydrosoluble, comprenant :
- un support granulaire neutre enrobé par une couche active comprenant :
. du diltiazem ou un sel pharmaceutiquement acceptable de ce dernier en tant que principe actif,
. un agent tensio-actif, et
. un agent liant,
- et une seule couche externe assurant la libération prolongée du principe actif (couche LP) et comprenant un polymère filmogène insoluble dans l'eau.

2. Microgranules selon la revendication 1, **caractérisés en ce que** la couche LP assure une libération prolongée lente du principe actif.

3. Microgranules selon la revendication 1, **caractérisés en ce que** la couche LP assure une libération prolongée rapide du principe actif.

4. Microgranules selon la revendication 2, **caractérisés en ce que** la couche LP assurant la libération prolongée lente du principe actif est enrobée à son tour par une autre couche active comprenant :
. du diltiazem ou un sel pharmaceutiquement acceptable de ce dernier en tant que principe actif,
. un agent tensio-actif, et
. un agent liant,
elle-même enrobée d'une couche externe assurant une libération prolongée rapide du principe actif contenu dans cette couche active.

5. Microgranules selon la revendication 4, **caractérisés en ce qu'**ils comprennent en outre une couche intercalaire entre la couche LP assurant la libération lente du principe actif et la couche active enrobant cette couche LP.

6. Microgranules selon l'une des revendications 1 à 5, **caractérisés en ce que** l'agent tensio-actif est un tensio-actif anionique, non-ionique, cationique ou amphotère.

7. Microgranules selon la revendication 6, **caractérisés en ce que** l'agent tensio-actif est un tensio-actif anionique, en particulier choisi parmi les alkylsulfates de métal alcalin en C₁₀-C₂₀, de préférence le laurylsulfate de sodium, les alkylsulfonates de métal alcalin en C₁₀-C₂₀, ou encore les alkyl-benzènesulfonates de métal alcalin en C₁₀-C₂₀.

8. Microgranules selon l'une des revendications 1 à 7, **caractérisés en ce que** l'agent liant est constitué par tout agent liant phar maceutiquement acceptable, utile pour l'enrobage des supports granulaires neutres, en particulier des polymères pharmaceutiquement acceptables tels que les polyvinylpyrrolidones.

9. Microgranules selon l'une des revendications 1 à 8, **caractérisés en ce que** chaque couche active comprend également un agent plastifiant, de préférence un ester de l'acide phtalique.

10. Microgranules selon l'une des revendications 1 à 9, **caractérisés en ce que** le rapport pondéral principe actif / agent tensio-actif est compris entre 99/1 et 95/5, de préférence d'environ 98/2.

11. Microgranules selon l'une des revendications 1 à 10, **caractérisés en ce que** le rapport pondéral principe actif / agent liant est compris entre 99/1 et 90/10, de préférence voisin de 97/3.

12. Microgranules selon l'une des revendications 1 à 3, **caractérisés en ce qu'**ils comprennent pour le support et la couche active les éléments suivants, les pourcentages étant donnés en poids pour un total de 100:
- support granulaire neutre 20 - 25 %
- principe actif 70 - 75 %
- agent tensio-actif 0,5 - 5 %
- agent liant 0,5 - 10 %
- agent plastifiant 0 - 5 %.

13. Microgranules selon la revendication 4, **caractérisés en ce qu'**ils comprennent pour le support et les couches actives les éléments suivants, les pourcentages étant donnés en poids pour un total de 100 :
- support granulaire neutre 10 - 20 %
- principe actif 75 - 85 %.
- agent tensio-actif 0,5 - 5 %
- agent liant 0,5 - 10 %
- agent plastifiant 0,5 - 5 %.

14. Microgranules selon l'une des revendications 1 à 13, **caracterisés en ce que** chaque couche LP est constituée par un agent d'enrobage assurant la libération prolongée, éventuellement associé à un ou plusieurs additifs usuels, en particulier un adjuvant de biodisponibilité et/ou un agent plastifiant et/ou un agent lubrifiant.

15. Microgranules selon la revendication 14, **caractérisés en ce que** l'agent d'enrobage assurant la libération prolongée est choisi parmi les polyméthacrylates.

16. Microgranules selon l'une des revendications 14 ou 15, **caractérisés en ce que** l'adjuvant de biodisponibilité est de préférence un ester d'acide gras et de polyoxyéthylène, en particulier ceux décrits sous le nom de polysorbate.

17. Microgranules selon l'une des revendications 14 à 16, **caractérisés en ce que** l'agent lubrifiant est du talc.

18. Microgranules selon l'une des revendications 14 à 17, **caractérisés en ce que** l'agent plastifiant est un agent plastifiant usuel pharmaceutiquement acceptable employé pour la préparation de microgranules, en particulier les esters des acides citrique, phtalique et cébacique, tels que le citrate de triéthyle, le sébaçate de dibutyle ou le phtalate de diéthyle, et leurs mélanges.

19. Microgranules selon l'une des revendications 1 à 3, **caractérisés en ce qu'**ils ont la composition finale suivante, les pourcentages étant exprimés en poids sec pour un total de 100 % :
- support granulaire neutre 10 - 20 %
• couche active :
- principe actif 45 - 65 %
- agent liant 0,5 - 2 %
- agent tensio-actif 0,5 - 1 %
- agent plastifiant 0,5 - 1 %
• couche LP :
- agent d'enrobage 10 - 30 %
- adjuvant de biodisponibilité 0,05 - 0,15 %
- agent plastifiant 2 - 10 %
- agent lubrifiant 2 - 10 %.

20. Microgranules selon la revendication 4, **caractérisés en ce qu'**ils ont la composition finale suivante, les pourcentages étant exprimés en poids sec pour un total de 100 % :
- support granulaire neutre 5 - 15 %
• couches actives :
- principe actif 45 - 60 %
- agent liant 0,5 - 2,5 %
- agent tensio-actif 0,75 - 1,25 %
- agent plastifiant 0,75 - 1,25 %
• couches LP :
- agent d'enrobage 15 - 35 %
- adjuvant de biodisponibilité 0,02 - 0,15 %
- agent plastifiant 2 - 10 %
- agent lubrifiant 2 - 10 %.

21. Gélules comprenant des microgranules selon l'une des revendications 1 à 20.

## Claims

1. Sustained-release (SR) microgranules containing Diltiazem and free from water-soluble organic acid, comprising:
- a neutral granular support coated with an active layer comprising:
· Diltiazem or a pharmaceutically acceptable salt thereof as active principle,
· a surfactant, and
· a binder,
- and a single external layer which ensures sustained release of the active principle (SR layer) and comprising a water-insoluble film-forming polymer.

2. Microgranules according to Claim 1, **characterized in that** the SR layer ensures slow sustained release of the active principle.

3. Microgranules according to Claim 1, **characterized in that** the SR layer ensures rapid sustained release of the active principle.

4. Microgranules according to Claim 2, **characterized in that** the SR layer which ensures slow sustained release of the active principle is itself coated with another active layer comprising:
· Diltiazem or a pharmaceutically acceptable salt thereof as active principle,
· a surfactant, and
· a binder,
itself coated with an external layer which ensures rapid sustained release of the active principle contained in this active layer.

5. Microgranules according to Claim 4, **characterized in that** they also comprise an intercalating layer between the SR layer which ensures slow release of the active principle and the active layer coating this SR layer.

6. Microgranules according to one of Claims 1 to 5, **characterized in that** the surfactant is an anionic, nonionic, cationic or amphoteric surfactant.

7. Microgranules according to Claim 6, **characterized in that** the surfactant is an anionic surfactant chosen, in particular, from alkali metal (C₁₀-C₂₀)alkyl sulphates, preferably sodium lauryl sulphate, alkali metal (C₁₀-C₂₀)alkyl sulphonates or alkali metal (C₁₀-C₂₀)alkyl benzenesulphonates.

8. Microgranules according to one of Claims 1 to 7, **characterized in that** the binder consists of any pharmaceutically acceptable binder which is useful for coating neutral granular supports, in particular pharmaceutically acceptable polymers such as polyvinylpyrrolidones.

9. Microgranules according to one of Claims 1 to 8, **characterized in that** each active layer also comprises a plasticizer, preferably a phthalic acid ester.

10. Microgranules according to one of Claims 1 to 9, **characterized in that** the active principle/surfactant weight ratio is between 99/1 and 95/5, preferably about 98/2.

11. Microgranules according to one of Claims 1 to 10, **characterized in that** the active principle/binder weight ratio is between 99/1 and 90/10, preferably in the region of 97/3.

12. Microgranules according to one of Claims 1 to 3, **characterized in that** they comprise the following components for the support and the active layer, the percentages being given on a weight basis for a total of 100:
- neutral granular support 20 - 25%
- active principle 70 - 75%
- surfactant 0.5 - 5%
- binder 0.5 - 10%
- plasticizer 0 - 5%

13. Microgranules according to Claim 4, **characterized in that** they comprise the following components for the support and the active layers, the percentages being given on a weight basis for a total of 100:
- neutral granular support 10 - 20%
- active principle 75 - 85%
- surfactant 0.5 - 5%
- binder 0.5 - 10%
- plasticizer 0.5 - 5%

14. Microgranules according to one of Claims 1 to 13, **characterized in that** each SR layer consists of a coating agent which ensures sustained release, optionally combined with one or more common additives, in particular a bioavailability adjuvant and/or a plasticizer and/or a lubricant.

15. Microgranules according to Claim 14, **characterized in that** the coating agent ensuring the sustained release is chosen from polymethacrylates.

16. Microgranules according to either of Claims 14 and 15, **characterized in that** the bioavailability adjuvant is preferably a fatty acid ester of polyoxyethylene, in particular those described under the name Polysorbate.

17. Microgranules according to one of Claims 14 to 16, **characterized in that** the lubricant is talc.

18. Microgranules according to one of Claims 14 to 17, **characterized in that** the plasticizer is a pharmaceutically acceptable common plasticizer used for the preparation of microgranules, in particular esters of citric, phthalic and sebacic acids, such as triethyl citrate, dibutyl sebacate or diethyl phthalate, and mixtures thereof.

19. Microgranules according to one of Claims 1 to 3, **characterized in that** they have the following final composition, the percentages being expressed on a dry weight basis for a total of 100%:
- neutral granular support 10 - 20%
· active layer:
- active principle 45 - 65%
- binder 0.5 - 2%
- surfactant 0.5 - 1%
- plasticizer 0.5 - 1%
· SR layer:
- coating agent 10 - 30%
- bioavailability adjuvant 0.05 - 0.15%
- plasticizer 2 - 10%
- lubricant 2 - 10%.

20. Microgranules according to Claim 4, **characterized in that** they have the following final composition, the percentages being expressed on a dry weight basis for a total of 100%:
- neutral granular support 5 - 15%
· active layers:
- active principle 45 - 60%
- binder 0.5 - 2.5%
- surfactant 0.75 - 1.25%
- plasticizer 0.75 - 1.25%
· SR layers:
- coating agent 15 - 35%
- bioavailability adjuvant 0.02 - 0.15%
- plasticizer 2 - 10%
- lubricant 2 - 10%

21. Gelatin capsules comprising microgranules according to one of Claims 1 to 20.

## Patentansprüche

1. Mikrogranalien mit verzögerter Freisetzung (VF), die Diltiazem enthalten und frei von wasserlöslicher organischer Säure sind, umfassend:
- einen neutralen granulären Träger, der von einer aktiven Schicht, die:
. Diltiazem oder ein pharmazeutisch annehmbares Salz des Letzteren als Wirkstoff,
. ein Tensid und
. ein Bindemittel,
umfasst,
- und von einer einzigen äußeren Schicht umhüllt ist, welche die verzögerte Freigabe des Wirkstoffes sicherstellt (VF-Schicht) und ein in Wasser unlösliches filmbildendes Polymer umfasst.

2. Mikrogranalien nach Anspruch 1, **dadurch gekennzeichnet, dass** die VF-Schicht eine langsame verzögerte Freisetzung des Wirkstoffes sicherstellt.

3. Mikrogranalien nach Anspruch 1, **dadurch gekennzeichnet, dass** die VF-Schicht eine schnelle verzögerte Freisetzung des Wirkstoffes sicherstellt.

4. Mikrogranalien nach Anspruch 2, **dadurch gekennzeichnet, dass** die VF-Schicht, welche die langsame verzögerte Freisetzung des Wirkstoffes sicherstellt, ihrerseits mit einer weiteren aktiven Schicht umhüllt ist, welche umfasst:
. Diltiazem oder ein pharmazeutisch annehmbares Salz des Letzteren als Wirkstoff,
. ein Tensid und
. ein Bindemittel,
welche selbst von einer äußeren Schicht umhüllt ist, die eine schnelle verzögerte Freisetzung des Wirkstoffes, der in dieser aktiven Schicht enthalten ist, sicherstellt.

5. Mikrogranalien nach Anspruch 4, **dadurch gekennzeichnet, dass** sie darüber hinaus eine Zwischenschicht zwischen der VF-Schicht, welche die langsame Freisetzung des Wirkstoffes sicherstellt, und der aktiven Schicht, welche diese VF-Schicht umhüllt, umfassen.

6. Mikrogranalien nach einem der Ansprüche 1 bis 5, **dadurch gekennzeichnet, dass** das Tensid ein anionisches, nicht-ionisches, kationisches oder amphoteres Tensid ist.

7. Mikrogranalien nach Anspruch 6, **dadurch gekennzeichnet, dass** das Tensid ein anionisches Tensid ist, das insbesondere aus (C₁₀-C₂₀)-Alkalimetallalkylsulfaten, vorzugsweise Natriumlaurylsulfat, (C₁₀-C₂₀)-Alkalimetallalkylsulfonaten oder auch (C₁₀-C₂₀)-Alkalimetallalkylbenzolsulfonaten ausgewählt ist.

8. Mikrogranalien nach einem der Ansprüche 1 bis 7, **dadurch gekennzeichnet, dass** das Bindemittel aus allen pharmazeutisch annehmbaren Bindemitteln zusammengesetzt ist, die für die Umhüllung von neutralen granulären Trägern geeignet sind, insbesondere aus pharmazeutisch annehmbaren Polymeren, wie Polyvinylpyrrolidonen.

9. Mikrogranalien nach einem der Ansprüche 1 bis 8, **dadurch gekennzeichnet, dass** jede aktive Schicht auch ein Plastifiziermittel umfasst, vorzugsweise einen Phthalsäureester.

10. Mikrogranalien nach einem der Ansprüche 1 bis 9, **dadurch gekennzeichnet, dass** das Gewichtsverhältnis Wirkstoff/Tensid zwischen 99/1 und 95/5 einschließlich, vorzugsweise etwa 98/2, eingeschlossen ist.

11. Mikrogranalien nach einem der Ansprüche 1 bis 10, **dadurch gekennzeichnet, dass** das Gewichtsverhältnis Wirkstoff/Bindemittel zwischen 99/1 und 90/10 einschließlich, vorzugsweise etwa 97/3, eingeschlossen ist.

12. Mikrogranalien nach einem der Ansprüch 1 bis 3, **dadurch gekennzeichnet, dass** sie für den Träger und die aktive Schicht die folgenden Bestandteile umfassen, wobei die Prozentsätze bezüglich Gewicht bei einer Summe von 100 angegeben sind:
- neutraler granulärer Träger 20 - 25%
- Wirkstoff 70 - 75%
- Tensid 0,5 - 5%
- Bindemittel 0,5 - 10%
- Plastifiziermittel 0 - 5%.

13. Mikrogranalien nach Anspruch 4, **dadurch gekennzeichnet, dass** sie für den Träger und die aktiven Schichten die folgenden Bestandteile umfassen, wobei die Prozentsätze bezüglich Gewicht bei einer Summe von 100 angegeben sind:
- neutraler granulärer Träger 10 - 20%
- Wirkstoff 75 - 85%
- Tensid 0,5 - 5%
- Bindemittel 0,5 - 10%
- Plastifiziermittel 0 - 5%.

14. Mikrogranalien nach einem der Ansprüche 1 bis 13, **dadurch gekennzeichnet, dass** jede VF-Schicht aus einem Umhüllungsmittel aufgebaut ist, welches die verzögerte Freisetzung sicherstellt, gegebenenfalls in Verbindung mit einem oder mehreren üblichen Zusätzen, insbesondere einem Bioverfügbarkeits-Adjuvans und/oder einem Plastifiziermittel und/oder einem Gleitmittel.

15. Mikrogranalien nach Anspruch 14, **dadurch gekennzeichnet, dass** das Umhüllungsmittel, das die verzögerte Freisetzung sicherstellt, aus Polymethacrylaten ausgewählt ist.

16. Mikrogranalien nach einem der Ansprüche 14 oder 15, **dadurch gekennzeichnet, dass** das Bioverfügbarkeits-Adjuvans vorzugsweise ein Polyoxyethylenfettsäureester ist, insbesondere diejenigen, die unter dem Namen Polysorbat beschrieben sind.

17. Mikrogranalien nach einem der Ansprüche 14 bis 16, **dadurch gekennzeichnet, dass** das Gleitmittel Talk ist.

18. Mikrogranalien nach einem der Ansprüche 14 bis 17, **dadurch gekennzeichnet, dass** das Plastifiziermittel ein übliches pharmazeutisch annehmbares Plastifiziermittel ist, das für die Herstellung von Mikrogranalien verwendet wird, insbesondere Citronensäure-, Phthalsäure- und Sebacinsäureester, wie Triethylcitrat, Dibutylsebacat oder Diethylphthalat und deren Mischungen.

19. Mikrogranalien nach einem der Ansprüche 1 bis 3, **dadurch gekennzeichnet, dass** sie die folgende Endzusammensetzung aufweisen, wobei die Prozentsätze als Trockengewicht bei einer Summe von 100% ausgedrückt sind:
- neutraler granulärer Träger 10- 20%
• aktive Schicht:
- Wirkstoff 45 - 65%
- Bindemittel 0,5 - 2%
- Tensid 0,5 - 1%
- Plastifiziermittel 0,5 - 1%
• VF-Schicht:
- Umhüllungsmittel 10 - 30%
- Bioverfügbarkeits-Adjuvans 0,05 - 0,15%
- Plastifiziermittel 2 - 10%
- Gleitmittel 2 - 10%.

20. Mikrogranalien nach Anspruch 4, **dadurch gekennzeichnet, dass** sie die folgende Endzusammensetzung aufweisen, wobei die Prozentsätze als Trockengewicht bei einer Summe von 100% ausgedrückt sind:
- neutraler granulärer Träger 5 - 15%
• aktive Schichten:
- Wirkstoff 45 - 60%
- Bindemittel 0,5 - 2,5%
- Tensid 0,75 - 1,25%
- Plastifiziermittel 0,75 - 1,25%
• VF-Schichten:
- Umhüllungsmittel 15 - 35%
- Bioverfügbarkeits-Adjuvans 0,02 - 0,15%
- Plastifiziermittel 2 - 10%
- Gleitmittel 2 - 10%.

21. Kapseln, umfassend die Mikrogranalien nach einem der Ansprüche 1 bis 20.
